# EUROPÄISCHE PATENTANMELDUNG

(11) **EP 4 008 713 A1**
(43) Veröffentlichungstag der Anmeldung: **08.06.2022**
(21) Anmeldenummer: 20211107.6
(22) Anmeldetag: 01.12.2020
(51) Int. Cl.: C07C 271/22, C07F 7/08, C07K 1/06

(54) **IODTYROSIN-DERIVATE UND VERFAHREN ZUR HERSTELLUNG VON IODTYROSIN-DERIVATEN**

(71) Anmelder: ABX Advanced Biochemical Compounds GmbH, 01454 Radeberg (DE)
(72) Erfinder: HOEPPING, Alexander, 01454 Radeberg (DE); MEYER, Christoph, 01454 Radeberg (DE); JOSEPH, Desna, 01454 Radeberg (DE); KÖSTER, Stefan David, 01454 Radeberg (DE); EISELT, erik, 01454 Radeberg (DE)
(74) Vertreter: Riechelmann & Carlsohn Patentanwälte PartG mbB

(57) **Zusammenfassung**

Die Erfindung betrifft eine Verbindung der allgemeinen Formel I 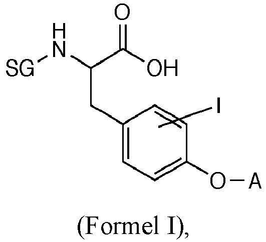 worin
A aus der Gruppe ausgewählt ist, die aus einer unverzweigten oder verzweigten Alkylgruppe mit 1 bis 12 Kohlenstoffatomen, einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R⁶)(O-R⁷)-Gruppe, einer -R¹-O-C(O)-O-R⁸-Gruppe besteht;
SG eine Schutzgruppe ist;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist; und
R⁹ ein zweiwertiger Kohlenwasserstoffrest mit 1 bis 12 Kohlenstoffatomen ist.

## Beschreibung

Die Erfindung betrifft Iodtyrosin-Derivate, insbesondere Fmoc-3-Iod-Tyrosinderivate und Boc-3-Iod-Tyrosinderivate. Sie betrifft ferner ein Verfahren zur Herstellung von Iodtyrosin-Derivaten, insbesondere von Fmoc-3-Iod-Tyrosinderivaten und Boc-3-Iod-Tyrosinderivaten. Sie betrifft außerdem die Verwendung von Iodtyrosin-Derivaten, insbesondere von Fmoc-3-Iod-Tyrosinderivaten und Boc-3-Iod-Tyrosinde-rivaten, bei der Synthese von Peptiden.

Die Modifikation von Peptiden mit 3-Iodtyrosin (D/L) dient im Wesentlichen der positiven Beeinflussung der Eigenschaften der Iodtyrosin tragenden Peptide. In vielen Fällen wird Iodtyrosin hauptsächlich am *N*-terminalen Ende von Peptiden oder kleinen Molekülen eingeführt [4]. Aufgrund der lipophilen Natur von Iodtyrosin verbessern sich die Bindungseigenschaften, was häufig zu verbesserten Rezeptoraffinitäten führt. Beispiele von Peptiden oder Peptidverbindungen, die Iodtyrosin enthalten, sind das theranostische Peptidpaar Pentixather/Pentixafor [5], HA-DOTA-TATE [6] oder PSMA I&T [7]. Ferner führt die Substitution von Tyrosin durch Iodtyrosin zu verbesserten Eigenschaften von z. B. Hormonen [8].

In den meisten Fällen wird Iodtyrosin unter Verwendung der kommerziell erhältlichen Bausteine Fmoc-3-Iod-L-tyrosin, Fmoc-3-Iod-D-tyrosin, Boc-3-Iod-D-Tyrosin oder Boc-3-Iod-L-Tyrosin eingeführt. Die Angabe "Fmoc" bezeichnet dabei die Schutzgruppe Fluorenylmethyloxycarbonyl. Die Angabe Boc bezeichnet die Schutzgruppe *tert*-Butyloxycarbonyl. Die Verwendung von Fmoc-3-Iod-L-tyrosin, Fmoc-3-Iod-D-tyrosin, Boc-3-Iod-D-Tyrosin oder Boc-3-Iod-L-Tyrosin kann jedoch mit unerwünschten Nebenreaktionen verbunden sein, da ihre Hydroxyfunktionalität in paraStellung noch immer ausreichend nukleophil ist, dass sie durch C-terminal aktivierte Aminosäuren acyliert werden kann. In den meisten Fällen führt dies zum Verlust der Aminosäure, die nicht länger für die Kopplung zur Verfügung steht. In diesem Zusammenhang können sich Kopplungen mit Fmoc-3-Iod-L-Tyrosin, Fmoc-3-Iod-D-Tyrosin, Boc-3-Iod-D-Tyrosin oder Boc-3-Iod-L-Tyrosin, bedingt durch die hohe Reaktivität der nukleophilen Hydroxyfunktionalität von Tyrosin, als nicht durchführbar erweisen, wenn es nur einen geringen oder keinen Umsatz in diesem Reaktionsschritt gibt.

Ein Weg, diesen Nachteil zu überwinden, ist die Iodierung von Tyrosin in dem Peptidendprodukt. Diese Verfahrensweise führt typischerweise zu zwei unterschiedlichen Produkten, die aus einem monoiodierten Tyrosin-Rest und einem diiodierten Tyrosin-Rest bestehen [9]. Beide müssen mittels Umkehrphasenchromatographie getrennt werden, was eine industrielle Anwendung erschwert. Ferner ist diese Methode nicht geeignet, wenn mehr als eine Tyrosin-Einheit in dem Peptid vorliegt. Im Weiteren eignet sich diese Methode nicht für eine industrielle Anwendung, da der Synthesemaßstab deutlich begrenzt ist. Zusätzlich muss der Ausgangsstoff abgetrennt werden.

Cobb et al. [10] schlagen die direkte Iodierung von vollständig geschütztem Fmoc-Tyr(tBu)-OH in Gegenwart von Ag₂SO₄ in Methanol vor, was hauptsächlich zu Fmoc-3-Iod-Tyr(tBu)-OMe führt, gefolgt von Verseifung. Diese Methode wird für größere Maßstäbe, wie sie in industriellen Anwendungen notwendig sind, als ungeeignet betrachtet. Amedio et al. [11] stellten die Verwendung von Boc-3-Iod-Tyr(PMB)-OH vor. In einem weiteren Beispiel verwendeten Kiyoyuki et al. Boc-3-Iod-Tyr(Boc)-OH für die Synthese eines cyclischen Peptids [12]. Beide geschützten Iodtyrosinderivate sind ausschließlich für die Boc-Chemie geeignet. Martiny et al. synthetisierten Fmoc-3-Iod-Tyr(TBDMS)-OH, was sich für die Einführung iodierter Tyrosine in Peptide mittels Fmoc/tBu-Chemie als geeignet erwies [13]. Der Nachteil dieser speziellen Verbindung ist die Anfälligkeit in Bezug auf (schwache) Säuren (z. B. Hexafluorisopropanol (HFIP)). Diese Anfälligkeit kann dazu führen, dass es nicht gelingt, die Peptide vollständig geschützt von dem Harz (z. B. unter Verwendung von HFIP) abzuspalten. Die Angabe "tBu" bezeichnet dabei die Schutzgruppe "*tert*-Butyl", die Angabe "Me" Methyl, die Angabe "boc" die Schutzgruppe *tert*-Butoxycarbonyl, die Angabe "PMB" die Schutzgruppe p-Methoxybenzyl, die Angabe "TBDMS" die Schutzgruppe *"tert-*Butyldimethylsilyl".

Aufgabe der Erfindung ist es, die Nachteile nach dem Stand der Technik zu beseitigen. Es sollen Iodtyrosin-Derivate, insbesondere Fmoc-3-Iod-Tyrosinderivate und Boc-3-Iod-Tyrosinderivate, angegeben werden, die eine Modifikation von Peptiden durch Einführung einer Iod-Tyrosin-Einheit ohne die beschriebenen Nebenreaktionen und die Abspaltung von derart modifizierten Peptiden im vollständig geschützten Zustand von einem Harz nicht behindern.

Diese Aufgabe wird durch die Merkmale der Ansprüche 1, 9 und 12 gelöst. Zweckmäßige Ausgestaltungen der Erfindungen ergeben sich aus den Merkmalen der Unteransprüche.

Nach Maßgabe der Erfindung ist eine Verbindung der allgemeinen Formel I 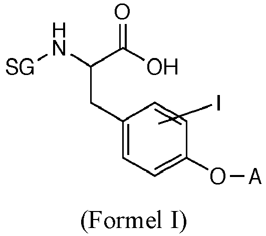 vorgesehen, worin
A aus der Gruppe ausgewählt ist, die aus einer unverzweigten oder verzweigten Alkylgruppe mit 1 bis 12 Kohlenstoffatomen, einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R⁶)(O-R⁷)-Gruppe, einer -R¹-O-C(O)-O-R⁸-Gruppe besteht;
SG eine Schutzgruppe ist;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist; und R⁹ ein zweiwertiger Kohlenwasserstoffrest mit 1 bis 12 Kohlenstoffatomen ist.

Die Schutzgruppe SG ist vorzugsweise aus der Gruppe ausgewählt, die aus einer Fluorenylmethyloxycarbonyl-Gruppe (Fmoc), einer *tert*-Butoxycarbonyl-Gruppe (Boc) und einer Benzyloxycarbonyl-Gruppe besteht. Stärker bevorzugt ist die Schutzgruppe SG Fluorenylmethyloxycarbonyl (Fmoc) oder *tert*-Butoxycarbonyl. Besonders bevorzugt ist die Schutzgruppe SG Fluorenylmethyloxycarbonyl (Fmoc). Die Schutzgruppe SG dient zum Schutz der Aminofunktion der Tyrosin-Einheit. Die Verbindungen der allgemeinen Formel I werden im Folgenden auch als SG-Iod-Tyrosine bezeichnet.

Eine Verbindung der allgemeinen Formel I, in der SG eine Fluorenylmethyloxycarbonyl-Gruppe (Fmoc) ist, ist eine Verbindung der allgemeinen Formel Ia; eine Verbindung der allgemeinen Formel I, in der SG eine *tert*-Butoxycarbonyl-Gruppe (Boc) ist, ist eine Verbindung der allgemeinen Formel Ib:

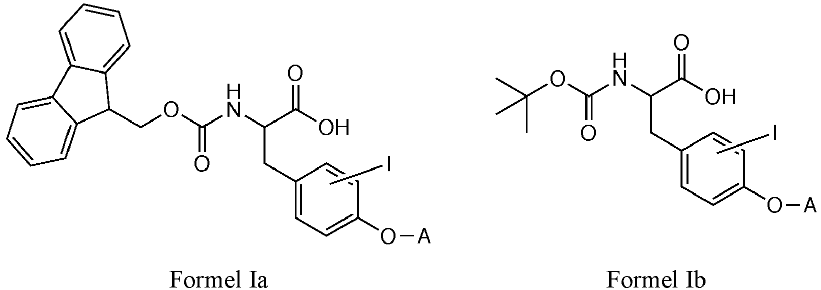

Die Verbindungen der allgemeinen Formel Ia werden im Folgenden auch als Fmoc-Iod-Tyrosine bezeichnet. Die Verbindungen der allgemeinen Formel Ib werden im Folgenden auch als Boc-Iod-Tyrosine bezeichnet.

Die erfindungsgemäße Verbindung der allgemeinen Formel I umfasst sowohl jedes der Enantiomere für sich als auch Gemische dieser Enantiomere. Die Tyrosineinheit der Verbindung der allgemeinen Formel I kann somit in D-Konfiguration, in L-Konfiguration oder als Gemisch von D- und L-Konfiguration vorliegen. Die Angabe "D/L" bezeichnet eine Verbindung, die in D-Konfiguration, in L-Konfiguration oder als Gemisch von D- und L-Konfiguration vorliegt.

Die erfindungsgemäße Verbindung der allgemeinen Formel I weist ein Iodatom auf, das an die Phenylgruppe der Tyrosineinheit gebunden ist.

Die erfindungsgemäßen Verbindungen ermöglichen die Einführung von SG-3-Iod-D-tyrosin(A)-OH oder SG-3-Iod-L-tyrosin(A)-OH in Peptide. Der Schutz der phenolischen Hydroxygruppe durch die Schutzgruppe A verhindert die unerwünschten Nebenreaktionen, die nach dem Stand der Technik mit der ungeschützten Hydroxyfunktionalität verbunden sind. Die Einheit A verhindert damit eine Acylierung durch C-terminal aktivierte Aminosäuren. Ein Verlust von Aminosäure wird damit verhindert. Außerdem sind nun schwierige Kopplungen mit SG-3-Iod-D-tyrosin(A)-OH oder SG-3-Iod-L-tyrosin(A)-OH durchführbar, weil die phenolische Hydroxyfunktionalität mittels der Einheit A geschützt ist. Die Erfindung ermöglicht insbesondere die Einführung von Fmoc-3-Iod-D-tyrosin(A)-OH oder Fmoc-3-Iod-L-tyrosin(A)-OH in Peptide. Sie ermöglicht ferner die Einführung von Boc-3-Iod-D-tyrosin(A)-OH oder Boc-3-Iod-L-tyrosin(A)-OH in Peptide. Die Angabe "(A)-OH" soll erkennen lassen, dass die phenolische Hydroxygruppe der Tyrosineinheit durch die Schutzgruppe A geschützt ist, die Hydroxygruppe der Carboxygruppe aber nicht geschützt ist. Nach der Einführung von SG-3-Iod-D-tyrosin(A)-OH oder SG-3-Iod-L-tyrosin(A)-OH in ein Peptid kann die Schutzgruppe A abgespalten werden.

Es kann vorgesehen sein, dass die Verbindung der allgemeinen Formel I eine Verbindung der allgemeinen Formel I-A 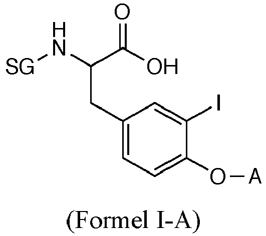 ist, worin A die in Anspruch 1 genannten Bedeutungen aufweist. Die Verbindung der allgemeinen Formel IA entspricht der Verbindung der allgemeinen Formel I, außer dass sich das Iodatom in 3-Stellung befindet. Eine Verbindung der allgemeinen Formel I-A, in der SG eine Fluorenylmethyloxycarbonyl-Gruppe (Fmoc) ist, ist eine Verbindung der allgemeinen Formel Ia-A; eine Verbindung der allgemeinen Formel I, in der SG eine *tert*-Butoxycarbonyl-Gruppe (Boc), ist eine Verbindung der allgemeinen Formel Ib-A:

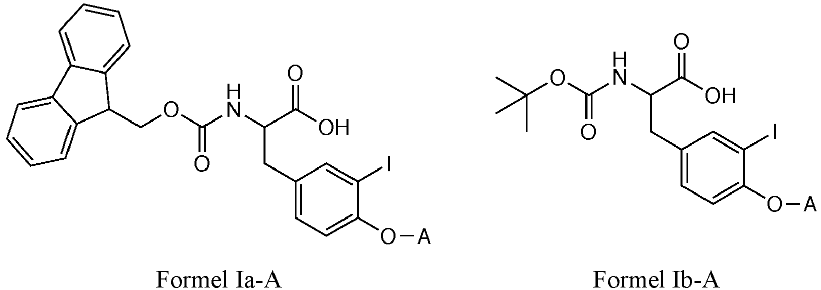

Die Einheit A ist eine Schutzgruppe zum Schutz der phenolischen Hydroxygruppe von SG-Iod-Tyrosin. Bei der Einheit A handelt es sich bevorzugt um eine Ethergruppe, eine Silylethergruppe, eine Acetalgruppe oder eine Carbonatgruppe. Im Falle von Fmoc-Iod-Tyrosin wird die Einheit A vorzugsweise so ausgewählt, dass sie mit der Fmoc/tBu-Strategie kompatibel ist, wie sie bei nicht-iodierten Fmoc-D/L-Tyrosin(tBu)-OH Anwendung findet. In Fmoc-D/L-Tyrosin(tBu)-OH ist die phenolische Hydroxyfunktionalität durch eine *tert*-Butylgruppe (tBu) geschützt. Die Einheit A ist außerdem so ausgewählt, dass eine Verwendung der erfindungsgemäßen Verbindung im Produktionsmaßstab möglich ist.

R¹ ist vorzugsweise eine unverzweigte Alkylengruppe mit 1 bis 6 Methyleneinheiten. Vorzugsweise ist R¹ Methylen, Ethylen oder *n*-Propylen.

R² ist vorzugsweise eine unverzweigte oder verzweigte Alkylgruppe mit 1 bis 12 Kohlenstoffatomen oder eine Arylgruppe, wobei eine unverzweigte oder verzweigte Alkylgruppe mit 1 bis 12 Kohlenstoffatomen bevorzugt ist.

Vorzugsweise sind R³, R⁴ und R⁵ unabhängig voneinander jeweils eine unverzweigte oder verzweigte Alkylgruppe mit 1 bis 2 Kohlenstoffatomen oder eine Arylgruppe.

Vorzugsweise sind R⁶ und R⁷ unabhängig voneinander jeweils eine unverzweigte oder verzweigte Alkylgruppe mit 1 bis 2 Kohlenstoffatomen oder eine Arylgruppe.

R⁸ ist vorzugsweise eine unverzweigte oder verzweigte Alkylgruppe mit 1 bis 12 Kohlenstoffatomen oder eine Arylgruppe, wobei eine unverzweigte oder verzweigte Alkylgruppe mit 1 bis 12 Kohlenstoffatomen bevorzugt ist.

R⁹ ist vorzugsweise eine unverzweigte Alkylengruppe mit 1 bis 6 Methyleneinheiten. Vorzugsweise ist R¹ Methylen, Ethylen, Propylen oder Butylen.

Es kann vorgesehen sein, dass die Einheit A aus der Gruppe ausgewählt ist, die aus einer unverzweigten oder verzweigten Alkylgruppe mit 1 bis 12 Kohlenstoffatomen, einer -R¹-O-R²-Gruppe, einer -R¹-Si(R³R⁴R⁵)-Gruppe und einer -C(O)-O-R⁹-Si(R³R⁴R⁵)-Gruppe besteht. Es kann ferner vorgesehen sein, dass die Einheit A aus der Gruppe ausgewählt ist, die aus
einer Alkylgruppe mit 1 bis 6 Kohlenstoffatomen; einer -R¹-O-R²-Gruppe, in der R¹ eine Alkylengruppe mit 1 bis 6 Kohlenstoffatomen ist und R² eine unverzweigte oder verzweigte Alkylgruppe mit 1 bis 6 Kohlenstoffatomen ist;
einer -R¹-Si(R³R⁴R⁵)-Gruppe, in der R¹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; und
einer -C(O)-O-R⁹-Si(R³R⁴R⁵)-Gruppe, in der R⁹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; besteht.

Bevorzugte Beispiele einer Verbindung der allgemeinen Formel Ia-A sind:
(i) 2-((((9*H*-Fluoren-9-yl)methoxy)carbonyl)amino)-3-(3-iod-4-(methoxymethoxy)phenyl)propionsäure, die auch als Fmoc-D/L-Tyr(MOM)-OH bezeichnet wird, wobei Fmoc-D-Tyr(MOM)-OH besonders bevorzugt ist;
(ii) 2-((((9*H*-Fluoren-9-yl)methoxy)carbonyl)amino)-3-(3-iod-4-(((2-(trimethylsilyl)ethoxy)carbonyl)oxy)phenyl)propionsäure, die auch als Fmoc-D/L-Tyr(TEOC)-OH bezeichnet wird, wobei Fmoc-D-Tyr(TEOC)-OH besonders bevorzugt ist;
(iii) 2-((((9*H*-Fluoren-9-yl)methoxy)carbonyl)amino)-3-(4-(2-(*tert*-butyldiphenylsilyl)ethoxy)-3-iodphenyl)propionsäure, die auch als Fmoc-D/L-Tyr(TBDPSE)-OH bezeichnet wird, wobei Fmoc-D-Tyr(TBDPSE)-OH besonders bevorzugt ist; und
(iv) 2-((((9*H*-Fluoren-9-yl)methoxy)carbonyl)amino)-3-(4-(*tert*-butoxy)-3-iodphenyl)propionsäure, das auch als Fmoc-D/L-Tyr(tBu)-OH bezeichnet wird, wobei Fmoc-D-Tyr(tBu)-OH besonders bevorzugt ist.

Bevorzugte Beispiele einer Verbindung der allgemeinen Formel Ib-A sind:
(i) 2-((*tert*-Butoxycarbonyl)amino)-3-(3-iod-4-(methoxymethoxy)phenyl)propionsäure, die auch als Boc-D/L-Tyr(MOM)-OH bezeichnet wird, wobei Boc-D-Tyr(MOM)-OH bevorzugt ist;
(ii) 2-((*tert*-Butoxycarbonyl)amino)-3-(3-iod-4-(((2-(trimethylsilyl)ethoxy)carbonyl)oxy)phenyl)propionsäure, die auch als Boc-D/L-Tyr(TEOC)-OH bezeichnet wird, wobei Boc-D-Tyr(TEOC)-OH bevorzugt ist;
(iii) 2-((*tert*-Butoxycarbonyl)amino)-3-(4-(2-(*tert*-butyldiphenylsilyl)ethoxy)-3-iodphenyl)propionsäure, die auch als Boc-D/L-Tyr(TBDPSE)-OH bezeichnet wird, wobei Boc-D-Tyr(TBDPSE)-OH bevorzugt ist; und
(iv) 2-((*tert*-Butoxycarbony)amino)-3-(4-(*tert*-butoxy)-3-iodphenyl)propionsäure, das auch als Boc-D/L-Tyr(tBu)-OH bezeichnet wird, wobei Boc-D-Tyr(tBu)-OH bevorzugt ist.

Nach Maßgabe der Erfindung ist ferner ein Verfahren zur Herstellung der erfindungsgemäßen Verbindung der allgemeinen Formel I vorgesehen. Dazu wird eine Verbindung der allgemeinen Formel II 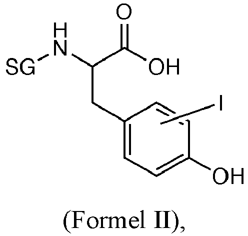 worin SG die in Zusammenhang mit der allgemeinen Formel I angegebenen Bedeutungen hat, mit einer Verbindung der allgemeinen Formel X-A, worin X Halogen oder Ammonium ist und A die in Zusammenhang mit der allgemeinen Formel I angegebenen Bedeutungen hat, zu einer Verbindung der allgemeinen Formel I 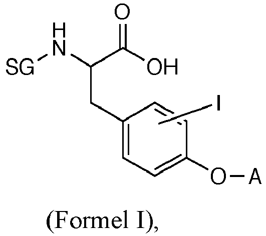 worin SG und A die im Zusammenhang mit Formel I angegebenen Bedeutungen aufweisen, umgesetzt. Mittels der Verbindung X-A wird die Schutzgruppe A in die Verbindung der allgemeinen Formel II eingebracht. Ist in der Verbindung der allgemeinen Formel II SG Fmoc, so handelt es sich bei dieser Verbindung um Fmoc-Iod-D/L-Tyrosin, das systematisch als 2-((((9*H*-Fluoren-9-yl)methoxy)carbonyl)amino)-3-(4-hydroxy-iodphenyl)propionsäure bezeichnet wird. Vorzugsweise ist die Verbindung der allgemeinen Formel II Fmoc-Iod-D-Tyrosin. Ist in der Verbindung der allgemeinen Formel II SG Boc, so handelt es sich bei dieser Verbindung um Boc-Iod-D/L-Tyrosin, das systematisch als 2-((*tert*-Butoxycarbonyl)amino)-3-(4-hydroxy-iodphenyl)propionsäure bezeichnet wird, wobei Boc-Iod-D-Tyrosin bevorzugt wird.

Eine besonders bevorzugte Verbindung der allgemeinen Formel II ist Fmoc-3-Iod-D/L-Tyrosin, das systematisch als 2-((((9*H*-Fluoren-9-yl)methoxy)carbonyl)amino)-3-(4-hydroxy-3-iodphenyl)propionsäure bezeichnet wird. In dieser Verbindung befindet sich das phenolische Iodatom in 3-Stellung. Besonders bevorzugt ist Fmoc-3-Iod-D-Tyrosin. Eine weitere bevorzugte Verbindung der allgemeinen Formel II ist Boc-3-Iod-D/L-Tyrosin, das systematisch als 2-((*tert-*Butoxycarbonyl)amino)-3-(4-hydroxy-3-iodphenyl)propionsäure bezeichnet wird. In dieser Verbindung befindet sich das phenolische Iodatom in 3-Stellung. Besonders bevorzugt ist Boc-3-Iod-D-Tyrosin.

Die Verbindung der allgemeinen Formel II kann aus einer Verbindung der allgemeinen Formel IV 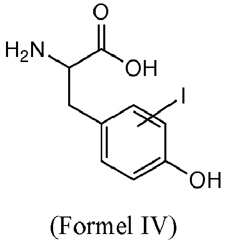 hergestellt werden. Die Aminfunktion der Verbindung der allgemeinen Formel IV wird durch die Einführung einer Schutzgruppe SG geschützt. Dazu kann die Verbindung der allgemeinen Formel IV beispielsweise mit einem 9-Fluorenylmethoxycarbonyl-Reagenz oder einem *tert*-Butoxycarbonyl-Reagenz umgesetzt werden. Bei der 9-Fluorenylmethoxycarbonyl-Verbindung kann es sich beispielsweise um (9-Fluorenylmethoxycarbonyloxy)-succinimid (Fmoc-OSu) handeln. Bei dem *tert*-Butoxycarbonyl-Reagenz kann es sich beispielsweise um Di-*tert*-butyldicarbonat (Boc₂O) handeln. Bei der Verbindung der allgemeinen Formel IV handelt es sich um D/L-Iodtyrosin, wobei D-Iodtyrosin bevorzugt ist.

Es kann vorgesehen sein, dass die Verbindung der allgemeinen Formel II mit einer Verbindung der allgemeinen der Formel X-A unter Erhalt einer Verbindung der allgemeinen Formel III 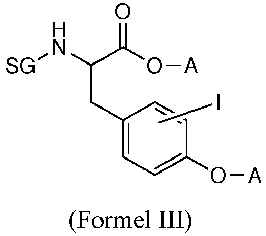 umgesetzt wird und die Verbindung der allgemeinen Formel III anschließend zu einer Verbindung der allgemeinen Formel I umgesetzt wird. Schema 1 veranschaulicht die erfindungsgemäße Herstellung einer erfindungsgemäßen Verbindung der allgemeinen Formel I aus einer Verbindung der allgemeinen Formel II. 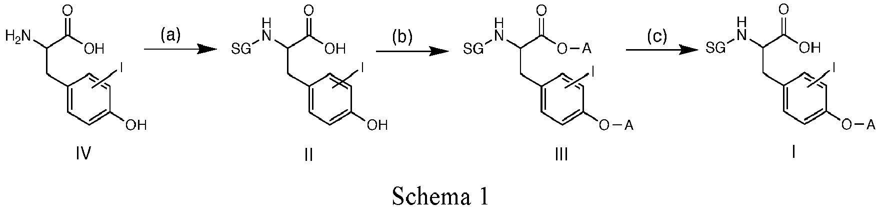 Schema 1a veranschaulicht die erfindungsgemäße Herstellung einer erfindungsgemäßen Verbindung der allgemeinen Formel Ia aus einer Verbindung der allgemeinen Formel IIa. Verbindung IIa ist eine Verbindung der allgemeinen Formel II, in der SG Fmoc ist. Das in Schema 1a gezeigte Verfahren ist eine Ausführungsform des in Schema 1 gezeigten Verfahrens. 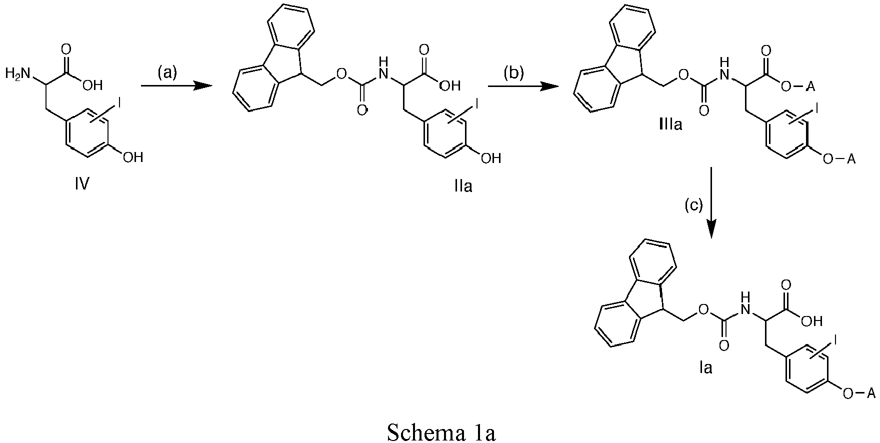 Schema 2 veranschaulicht die erfindungsgemäße Herstellung einer erfindungsgemäßen Verbindung der allgemeinen Formel Ia-A aus 3-Iod-D/L-Tyrosin. Das in Schema 2 gezeigte Verfahren ist eine bevorzugte Ausführungsform des in Schema 1a gezeigten Verfahrens.

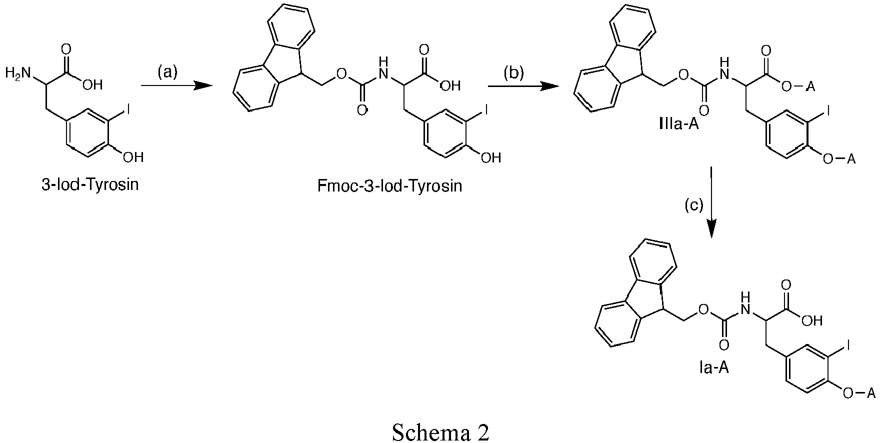

Schritt (a) des in Schema 1 gezeigten Verfahrens sieht die Umsetzung einer Verbindung der allgemeinen Formel IV zu einer Verbindung der allgemeinen Formel II vor. Dabei wird am N-Terminus der Verbindung der allgemeinen Formel IV eine Schutzgruppe zum Schutz der Aminfunktion eingeführt. Dazu kann die Verbindung der allgemeinen Formel IV beispielsweise mit (9-Fluorenylmethoxycarbonyloxy)-succinimid (Fmoc-OSu) (siehe Schema 1a) oder Di-*tert*-butyldicarbonat (Boc₂O) umgesetzt werden. Die Verbindung der allgemeinen Formel II entspricht der Verbindung der allgemeinen Formel IV, außer dass der N-Terminus der Verbindung der allgemeinen Formel IV durch eine Schutzgruppe SG geschützt ist. Unter Umgebungstemperatur wird eine Temperatur im Bereich von 18 bis 25 °C verstanden.

Soll in Schritt (a) von Schema 1 Fmoc als Schutzgruppe SG eingeführt werden, so kann Schritt (a) bei Umgebungsdruck und Umgebungstemperatur unter einem Schutzgas, beispielsweise unter Argon-Atmosphäre, durchgeführt werden. Zur Einführung von Fmoc als Schutzgruppe SG wird Schritt (a) in diesem Fall vorzugsweise in einem Gemisch aus einer wässerigen Natriumcarbonat-Lösung und 1,4-Dioxan durchgeführt.

Soll in Schritt (a) von Schema 1 Boc als Schutzgruppe SG eingeführt werden, so kann Schritt (a) bei Umgebungsdruck und Umgebungstemperatur an der Luft durchgeführt werden. Ein Schutzgas ist nicht erforderlich. Schritt (a) wird in diesem Fall vorzugsweise in einem Gemisch aus Wasser, Tetrahydrofuran und Triethylamin durchgeführt.

Schritt (b) des in Schema 1 gezeigten Verfahrens sieht die Umsetzung einer Verbindung der allgemeinen Formel II zu einer Verbindung der allgemeinen Formel III vor. Dabei werden die Hydroxygruppe am C-Terminus der Verbindung der allgemeinen Formel II und die phenolische Hydroxygruppe durch eine Einheit A geschützt. Dazu wird die Verbindung der allgemeinen Formel II mit der Verbindung X-A umgesetzt. Die Umsetzung kann in einem aprotischen Lösungsmittel wie Dichlormethan (DCM) in Gegenwart einer Hilfsbase wie Diisopropylethylamin (Hünig-Base, DIPEA) und eines Phasentransferkatalysators wie Tetrabutylammoniumchlorid (TBACl) stattfinden. Die Umsetzung kann in einem Temperaturbereich zwischen 0 °C und Umgebungstemperatur durchgeführt werden. Sie kann bei Umgebungsdruck und unter einem Schutzgas, beispielsweise unter Argonatmosphäre, durchgeführt werden. Die Verbindung der allgemeinen Formel III entspricht der Verbindung der allgemeinen Formel II, außer dass die Hydroxygruppe am C-Terminus der Verbindung der allgemeinen Formel II und die phenolische Hydroxygruppe durch die Einheit A geschützt sind.

Schritt (c) des in Schema 1 gezeigten Verfahrens sieht die Umsetzung einer Verbindung der allgemeinen Formel III zu einer Verbindung der allgemeinen Formel I vor. Dabei wird die Einheit A, die die Hydroxygruppe am C-Terminus der Verbindung der allgemeinen Formel III schützt, abgespalten, während die Einheit A, die die phenolische Hydroxygruppe schützt, erhalten bleibt. Die Abspaltung erfolgt im basischen Bereich, beispielsweise in einem Pyridin/Wasser Gemisch. Die Umsetzung kann in einem Temperaturbereich zwischen 0 °C und Umgebungstemperatur durchgeführt werden. Sie kann bei Umgebungsdruck durchgeführt werden. Ein Schutzgas ist nicht erforderlich. Die Verbindung der allgemeinen Formel I entspricht der Verbindung der allgemeinen Formel III, außer dass sich am C-Terminus der Verbindung der allgemeinen Formel I eine Hydroxygruppe befindet.

Weitere Einzelheiten des erfindungsgemäßen Verfahrens sind im Zusammenhang mit der erfindungsgemäßen Verbindung der allgemeinen Formel I bereits beschrieben worden. Auf deren Beschreibung wird verwiesen.

Nach Maßgabe der Erfindung ist eine Verwendung einer erfindungsgemäßen Verbindung der allgemeinen Formel I zur Herstellung eines Peptids vorgesehen. Das hergestellte Peptid weist zumindest eine Iod-Tyrosin-Einheit auf. Das hergestellte Peptid kann bekannten Peptiden entsprechen, abgesehen davon, dass zumindest eine, vorzugsweise genau eine Tyrosin-Einheit durch eine 3-Iod-Tyrosin-Einheit ersetzt ist. Die 3-Iod-Tyrosin-Einheit kann durch Reaktion einer Verbindung der allgemeinen Formel I mit einer Aminosäure oder einer Aminosäuresequenz unter Erhalt eines Peptides hergestellt werden. Die Herstellung des Peptids kann mittels an sich bekannter Syntheseverfahren erfolgen, beispielsweise mittels der Merrifield-Synthese. Ein Weg zur Peptidsynthese ist in Robert Bruce Merrifield, Solid phase peptide synthesis Journal of the American Chemical Society, Volume 85, Heft 14 S. 2149-2154 beschrieben. Nach der Herstellung des Peptids wird die Einheit A, die aus der Verbindung der allgemeinen Formel I stammt, abgespalten, wodurch ein Peptid mit einer 3-Iod-Tyrosin-Einheit erhalten wird, dessen phenolische OH-Gruppe ungeschützt ist.

Schema 3 veranschaulicht die Herstellung eines Peptides der allgemeinen Formel V, das eine Iod-Tyrosin-Einheit aufweist, unter Verwendung einer Verbindung der allgemeinen Formel I. 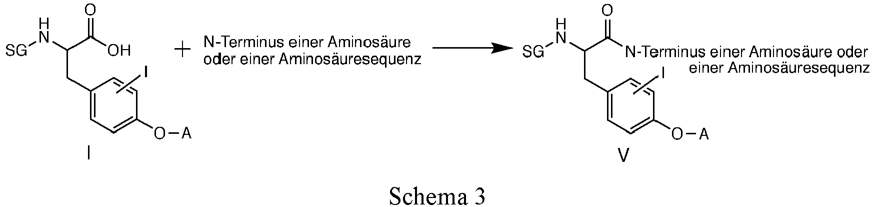 Schema 3a veranschaulicht die Herstellung eines Peptides der allgemeinen Formel Va, das eine Iod-Tyrosin-Einheit aufweist, unter Verwendung einer Verbindung der allgemeinen Formel Ia. Ein Peptid der allgemeinen Formel Va ist ein Peptid der allgemeinen Formel V, in der SG Fmoc ist. 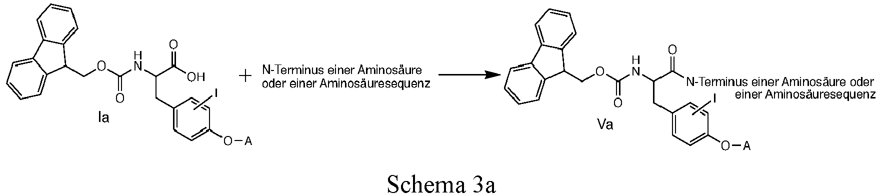 Anschließend kann die Schutzgruppe SG abgespalten werden, wodurch die Verbindung der allgemeinen Formel V in eine Verbindung der allgemeinen Formel VI überführt wird, wie in Schema 4 und Schema 4a gezeigt ist. 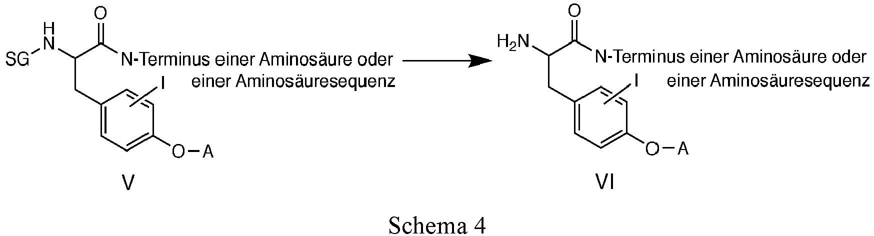 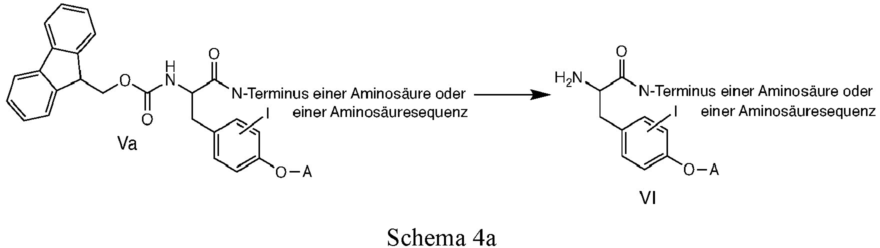 Soll die Iod-Tyrosin-Einheit keine terminale Einheit des Peptids sein, so kann eine weitere Aminosäure an den N-Terminus der Verbindung der allgemeinen Formel VI gekoppelt werden, wodurch, wie in Schema 5 gezeigt ist, eine Verbindung der allgemeinen Formel VII erhalten wird. 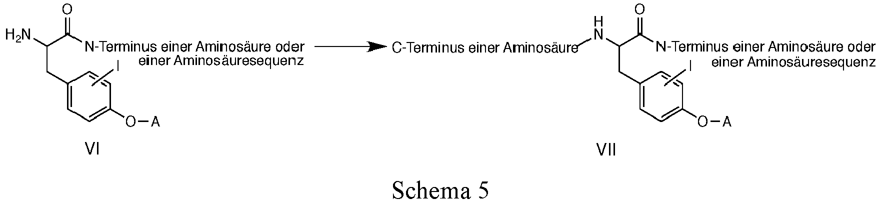 An die weitere Aminosäure können eine oder mehrere zusätzliche Aminosäuren gebunden werden, wodurch ein Peptid der allgemeinen Formel VIII 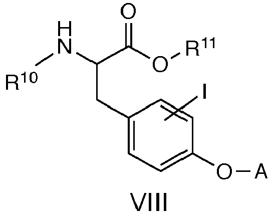 erhalten werden kann, in der A die in Zusammenhang mit der Verbindung der allgemeinen Formel I angegebenen Bedeutungen hat, R¹⁰ Wasserstoff oder eine oder mehrere Aminosäureeinheiten ist und R¹¹ Wasserstoff oder eine oder mehrere Aminosäureeinheiten ist, mit der Maßgabe, dass, wenn R¹⁰ Wasserstoff ist, R¹¹ nicht Wasserstoff ist und dass, wenn R¹¹ Wasserstoff ist, R¹⁰ nicht Wasserstoff ist. Bei einer Aminosäureeinheit kann es sich um eine Einheit handeln, die am N-Terminus eine NH-Gruppe oder eine NR¹²-Gruppe aufweist, wobei R¹² eine Methylgruppe ist.

Die Verbindung der allgemeinen Formel VIII kann durch Abspalten der Einheit A in ein Peptid der allgemeinen Formel IX überführt werden, wie in Schema 6 gezeigt ist. 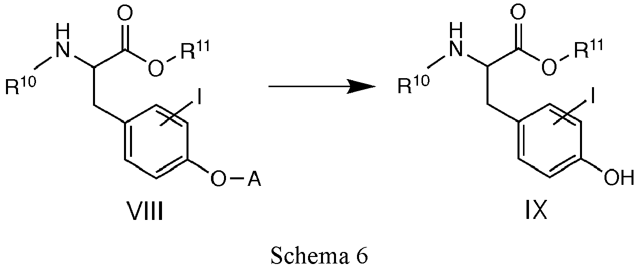 Die Abspaltung der Einheit A erfolgt im sauren Bereich, beispielsweise im Zuge der Totalentschützung des Peptids. Zur Totalentschützung des Peptids wird vorzugsweise eine wässerige Lösung von Trifluoressigsäure (TFA), beispielsweise eine 95%ige TFA-Lösung verwendet. Die Umsetzung kann in einem Temperaturbereich zwischen 0 °C und Umgebungstemperatur durchgeführt werden. Sie kann bei Umgebungsdruck durchgeführt werden. Ein Schutzgas ist nicht erforderlich. Die Verbindung der allgemeinen Formel IX entspricht der Verbindung der allgemeinen Formel VIII, außer dass die Einheit A unter Erhalt einer Hydroxygruppe abgespalten wurde.

Der Ausdruck "Alkyl" bezieht sich, sofern nichts anderes angegeben ist, insbesondere auf eine einwertige gesättigte aliphatische Kohlenwasserstoff-Gruppe mit einer verzweigten oder unverzweigten Kohlenstoffkette mit 1 bis 12 Kohlenstoffatomen, vorzugsweise 1 bis 8 Kohlenstoffatomen und besonders bevorzugt 1 bis 6 Kohlenstoffatomen. Beispiele von Alkylgruppen umfassen, sind aber nicht beschränkt auf Methyl, Ethyl, Propyl, Isopropyl, Isobutyl, *sec*-Butyl, *tert-Butyl,* Pentyl, *n*-Hexyl, Octyl, Dodecyl und dergleichen.

Der Ausdruck "Alkylen" bezieht sich, sofern nichts anderes angegeben ist, insbesondere auf eine zweiwertige gesättigte aliphatische Kohlenwasserstoff-Gruppe mit einer verzweigten oder unverzweigten Kohlenstoffkette mit 1 bis 12 Kohlenstoffatomen, vorzugsweise 1 bis 8 Kohlenstoffatomen und besonders bevorzugt 1 bis 6 Kohlenstoffatomen. Beispiele von Alkylengruppen umfassen, sind aber nicht beschränkt auf Methylen, Ethylen, Propylen, Butylen und dergleichen.

Der Ausdruck "Aryl" bezieht sich, sofern nichts anderes angegeben ist, auf eine cyclische, aromatische Kohlenwasserstoffgruppe, die aus einem mono-, bi- oder tricyclischen aromatischen Ringsystem mit 5 bis 18 Ringatomen, bevorzugt 5 oder 6 Ringatomen, besteht. Beispiele von Arylgruppen umfassen, sind aber nicht beschränkt auf Phenyl, Naphthyl, Anthracenyl, Phenanthryl, Fluorenyl, Indenyl, Azulenyl, Biphenyl, Methylendiphenyl und dergleichen, einschließlich teilweise hydrierte Derivate davon. Die Arylgruppe kann, sofern nichts anderes angegeben ist, ein- oder mehrwertig, beispielsweise ein- oder zweiwertig sein.

Die Erfindung wird nachstehend anhand von Ausführungsbeispielen, die die Erfindung nicht einschränken sollen, näher erläutert.

Beispiele erfindungsgemäßer Verbindungen sind in Tabelle 1 angegeben. Die Verbindungen **1D, 2D**, **3D** und **4D** besitzen eine R-Konfiguration und sind Derivate von D-Tyrosin. Die Verbindungen **1L, 2L, 3L** und **4L** besitzen eine S-Konfiguration und sind Derivate von L-Tyrosin.

**Tabelle 1:**

| Verbindung | Struktur | Name (Kurzname) |
|---|---|---|
| **1D** | 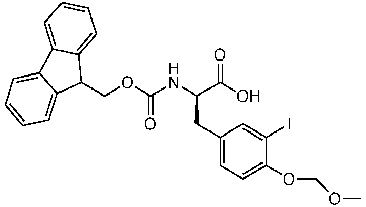 | (*R*)-2-((((9*H*-Fluoren-9-yl)methoxy)carbonyl)amino)-3-(3-iod-4-(methoxymethoxy)phenyl)propionsäure (Fmoc-3 -Iod-D-Tyr(MOM)-OH)) |
| **1L** | 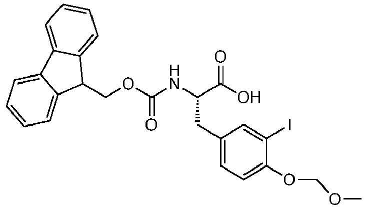 | (*S*)-2-((((9*H*-Fluoren-9-yl)methoxy)carbonyl)amino)-3-(3-iod-4-(methoxymethoxy)phenyl)propionsäure (Fmoc-3 -Iod-L-Tyr(MOM)-OH)) |
| **2D** | 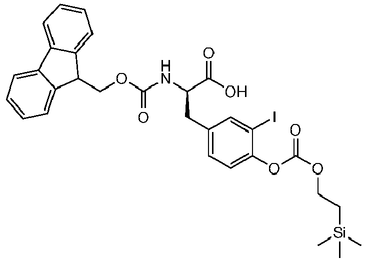 | (*R*)-2-((((9*H*-Fluoren-9-yl)methoxy)carbonyl)amino)-3-(3-iod-4-(((2-(trimethylsilyl)ethoxy)carbonyl)oxy)phenyl)propionsäure (Fmoc-3 -Iod-D-Tyr(TEOC)-OH) |
| **2L** | 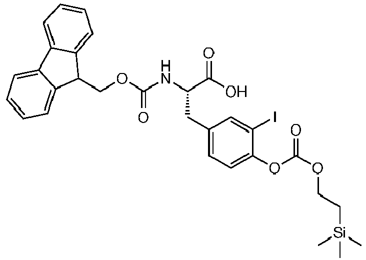 | (*S*)-2-((((9*H*-Fluoren-9-yl)methoxy)carbonyl)amino)-3-(3-iod-4-(((2-(trimethylsilyl)ethoxy)carbonyl)oxy)phenyl)propionsäure (Fmoc-3-Iod-L-Tyr(TEOC)-OH) |
| **3D** | 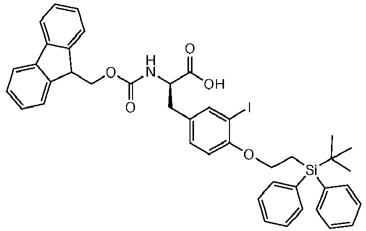 | (*R*)-2-((((9*H*-Fluoren-9-yl)methoxy)carbonyl)amino)-3-(4-(2-(*tert*-butyldiphenylsilyl)ethoxy)-3 -iodphenyl)propionsäure (Fmoc-3 -Iod-D-Tyr(TBDP SE)-OH) |
| **3L** | 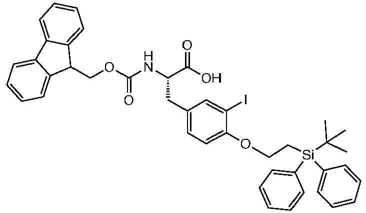 | (*S*)-2-((((9*H*-Fluoren-9-yl)methoxy)carbonyl)amino)-3-(4-(2-(*tert*-butyldiphenylsilyl)ethoxy)-3 -iodphenyl)propionsäure (Fmoc-3 -Iod-L-Tyr(TBDP SE)-OH) |
| **4D** | 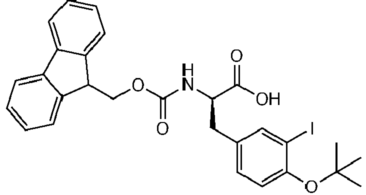 | (*R*)-2-((((9*H*-Fluoren-9-yl)methoxy)carbonyl)amino)-3-(4-(*tert-*butoxy)-3 -iodphenyl)propionsäure (Fmoc-3 -Iod-D-Tyr(tBu)-OH) |
| **4L** | 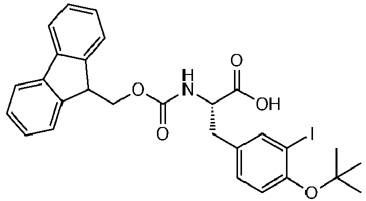 | (*S*)-2-((((9*H*-Fluoren-9-yl)methoxy)carbonyl)amino)-3-(4-(*tert-*butoxy)-3 -iodphenyl)propionsäure (Fmoc-3 -Iod-L-Tyr(tBu)-OH) |

Die in Tabelle 1 genannten Verbindungen sind beispielhafte Verbindungen der allgemeinen Formel I und der allgemeinen Formel Ia. Die Verbindungen **1D** und **1L** sind Verbindungen der allgemeinen Formel Ia, in denen A eine -R¹-O-R²-Gruppe ist, in der R¹ eine Methylengruppe und R² eine Methylgruppe ist. Die Verbindungen **2D** und **2L** sind Verbindungen der allgemeinen Formel Ia, in denen A eine -R¹-Si(R³R⁴R⁵)-Gruppe ist, in der R¹ -CH₂-CH₂- ist und R³, R⁴ und R⁵ jeweils eine Methylgruppe sind. Die Verbindungen **3D** und **3L** sind Verbindungen der allgemeinen Formel Ia, in denen A eine -R¹-Si(R³R⁴R⁵)-Gruppe ist, in der R¹ -CH₂-CH₂-CH₂- ist, R³ und R⁴ jeweils eine Phenylgruppe sind und R⁵ eine *tert*-Butylgruppe ist. Die Verbindungen **4D** und **4L** sind Verbindungen der allgemeinen Formel Ia, in denen A eine *tert*-Butylgruppe ist.

Weitere Beispiele erfindungsgemäßer Verbindungen sind in Tabelle 1a angegeben. Die Verbindungen **5D, 6D, 7D** und **8D** besitzen eine R-Konfiguration und sind Derivate von D-Tyrosin. Die Verbindungen **5L, 6L, 7L** und **8L** besitzen eine S-Konfiguration und sind Derivate von L-Tyrosin.

**Tabelle 1a:**

| Verbindung | Struktur | Name (Kurzname) |
|---|---|---|
| **5D** | 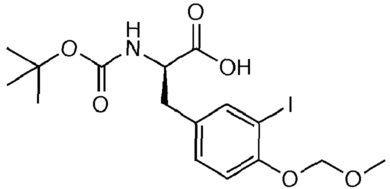 | (*R*)-2-((*tert*-Butoxycarbonyl)amino)-3-(3-iod-4-(methoxymethoxy)phenyl)propi onsäure (Boc-3-Iod-D-Tyr(MOM)-OH)) |
| **5L** | 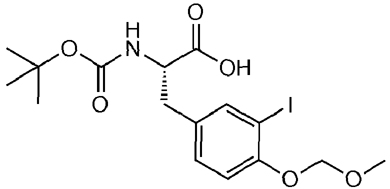 | (*S*)-2-((*tert*-Butoxycarbonyl)amino)-3 -(3 -iod-4-(methoxymethoxy)phenyl)propionsäure (Boc-3-Iod-L-Tyr(MOM)-OH)) |
| **6D** | 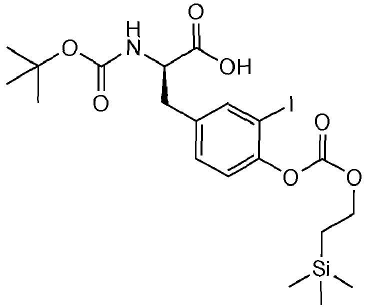 | (*R*)-2-((*tert*-Butoxycarbonyl)amino)-3 -(3 -iod-4-(((2-(trimethylsilyl)ethoxy)carbonyl)oxy)phenyl)propionsäure (Boc-3-Iod-D-Tyr(TEOC)-OH) |
| **6L** | 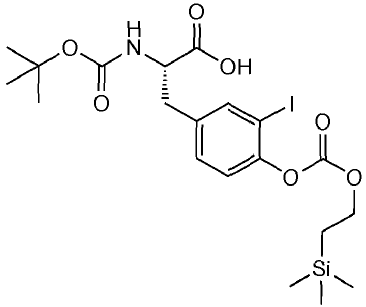 | (*S*)-2-((*tert*-Butoxycarbonyl)amino)-3 -(3 -iod-4-(((2-(trimethylsilyl)ethoxy)carbonyl)oxy)phenyl)propionsäure (Boc-3-Iod-L-Tyr(TEOC)-OH) |
| **7D** | 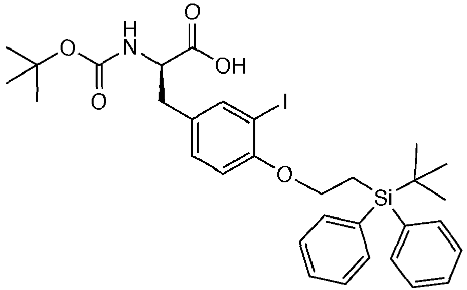 | (*R*)-2-((*tert*-Butoxycarbonyl)amino)-3-(4-(2-(*tert*-butyldiphenylsilyl)ethoxy)-3 -iodphenyl)propionsäure (Boc-3-Iod-D-Tyr(TBDPSE)-OH) |
| **7L** | 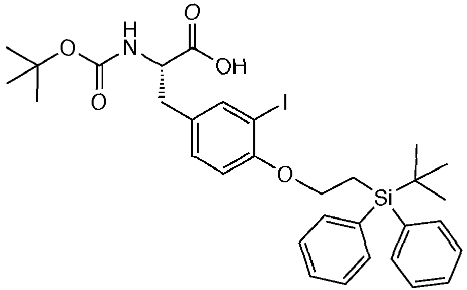 | (*S*)-2-((*tert*-Butoxycarbonyl)amino)-3-(4-(2-(*tert*-butyldiphenylsilyl)ethoxy)-3-iodphenyl)propionsäure (Boc-3-Iod-L-Tyr(TBDPSE)-OH) |
| **8D** | 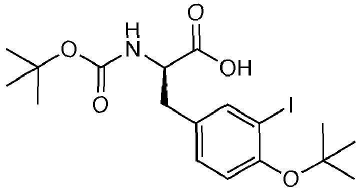 | (*R*)-2-((*tert*-Butoxycarbonyl)amino)-3-(4-(*tert-*butoxy)-3-iodphenyl)propionsäure (Boc-3-Iod-D-Tyr(tBu)-OH) |
| **8L** | 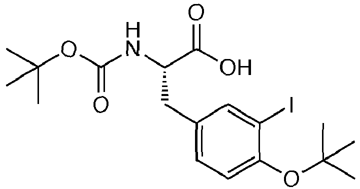 | (*S*)-2-((*tert*-Butoxycarbonyl)amino)-3-(4-(*tert-*butoxy)-3 -iodphenyl)propionsäure (Boc-3-Iod-L-Tyr(tBu)-OH) |

Die in Tabelle 1a genannten Verbindungen sind beispielhafte Verbindungen der allgemeinen Formel I und der allgemeinen Formel Ib. Die Verbindungen **5D** und **5L** sind Verbindungen der allgemeinen Formel Ib, in denen A eine -R¹-O-R²-Gruppe ist, in der R¹ eine Methylengruppe und R² eine Methylgruppe ist. Die Verbindungen **6D** und **6L** sind Verbindungen der allgemeinen Formel Ib, in denen A eine -R¹-Si(R³R⁴R⁵)-Gruppe ist, in der R¹ -CH₂-CH₂- ist und R³, R⁴ und R⁵ jeweils eine Methylgruppe sind. Die Verbindungen **7D** und **7L** sind Verbindungen der allgemeinen Formel Ib, in denen A eine -R¹-Si(R³R⁴R⁵)-Gruppe ist, in der R¹ -CH₂-CH₂-CH₂- ist, R³ und R⁴ jeweils eine Phenylgruppe sind und R⁵ eine *tert*-Butylgruppe ist. Die Verbindungen **8D** und **8L** sind Verbindungen der allgemeinen Formel Ib, in denen A eine *tert*-Butylgruppe ist.

Die in den Kurznamen verwendeten Abkürzungen haben folgende Bedeutung:
- Boc: *tert*-Butoxycarbonyl
- Fmoc: 9-Fluorenylmethyloxycarbonyl
- MOM: Methoxymethyl
- OH: Hydroxygruppe der Carboxyleinheit
- TBDPSE: *tert*-Butyldiphenylsilylethyl
- TEOC: 2-(Trimethylsilyl)ethoxycarbonyl
- D-Tyr: D-Tyrosin
- L-Tyr: L-Tyrosin

### Beispiel 1

### Synthese von Fmoc-3-Iod-D-Tyr(MOM)-OH (1D)

Die Synthese von Fmoc-3-Iod-D-Tyr(MOM)-OH wurde, wie in Schema B1 beschrieben, durchgeführt: 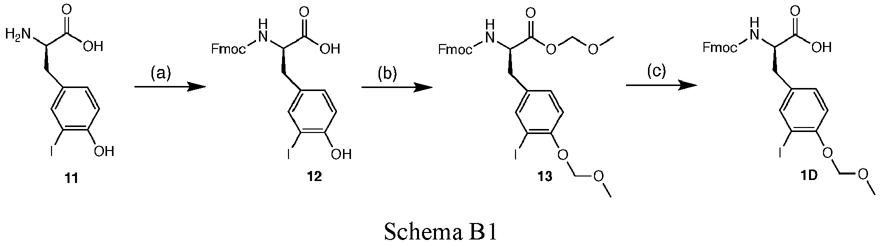 In Schritt (a) wird (*R*)-2-Amino-3-(4-hydroxy-3-iodphenyl)propionsäure **11** (die auch als 3-Iod-D-Tyrosin oder 3-Iod-D-Tyr-OH bezeichnet wird) mit *N*-(9-Fluorenylme-thoxycarbonyloxy)-succinimid (Fmoc-OSu) unter Erhalt von (*R*)-2-((((9*H*-Fluoren-9-yl)methoxy)carbonyl)amino)-3-(4-hydroxy-3-iodphenyl)propionsäure **12** (die auch als Fmoc-3-Iod-D-Tyr-OH bezeichnet wird) umgesetzt. Die Umsetzung findet in einem Gemisch aus einer wässerigen Natriumcarbonat-Lösung und 1,4-Dioxan statt. In Schritt (b) wird Verbindung **12** dann mit Methoxymethylbromid (CH₃-O-CH₂-Br) zu Methoxymethyl-(*R*)-2-((((9*H*-fluoren-9-yl)methoxy)carbonyl)amino)-3-(3-iod-4-(methoxy-methoxy)phenyl)propanoat **13** (das auch als Fmoc-3-Iod-D-Tyr(MOM)-OMOM bezeichnet wird) umgesetzt. Die Umsetzung findet in Dichlormethan (DCM) in Gegenwart von Diisopropylethylamin (DIPEA) und Tetrabutylammoniumchlorid (TBACl) statt. In Schritt (c) wird Verbindung **13** dann zur Zielverbindung **1D** umgesetzt. Die Umsetzung erfolgt in einem Gemisch aus Tetrahydrofuran (THF), Wasser und Pyridin.

Die Analyse der hergestellten Verbindung erfolgte sowohl durch HPLC-Analyse als auch durch LC-MS-Analyse.

### a) Synthese von Fmoc-3-Iod-D-Tyr-OH (12)

3-Iod-D-Tyr-OH **11** (5 g, 16,28 mmol) wurde in 50 ml wässeriger Na₂CO₃-Lösung (1,726 g, 16,28 mmol) in einer Argonatmosphäre suspendiert. 10 ml Dioxan wurden zugegeben, und die gelbe Lösung wurde in einem Eis-Wasser-Bad abgekühlt. Fmoc-OSu (5,492 g, 16,28 mmol), gelöst in 50 ml 1,4-Dioxan, wurde tropfenweise über einen Tropftrichter unter einer Argonatmosphäre zugegeben. Nach der Zugabe wurde das Reaktionsgemisch in einem Eis-Wasser-Bad für 1 h, dann bei Raumtemperatur gerührt. Nach 17 h zeigte sich in der Dünnschichtchromatographie (DC mit (DCM/Methanol (MeOH), 9:1 als Eluent) ein vollständiger Umsatz zu dem gewünschten Produkt Fmoc-3-Iod-D-Tyr-OH. 100 ml H₂O wurden zugegeben, und das Gemisch wurde in einem Eis-Wasser-Bad abgekühlt. 30 % HCl (ungefähr 4 ml) wurden zugegeben, bis ein pH von 2 bis 3 erreicht war. Das Gemisch wurde mit Ethylacetat (3 x 150 ml) extrahiert, die vereinigten organischen Phasen wurden mit H₂O (2 x 150 ml) und Salzlösung (1 x 150 ml) gewaschen, über Na₂SO₄ getrocknet und filtriert (Filter Porengröße 4). Das Lösungsmittel wurde durch Rotationsverdampfen entfernt, der Rest wurde unter Hochvakuum getrocknet. Ausbeute: 9,5 g (110 %, quant.) eines weißen schaumartigen Feststoffes. Das Rohprodukt wurde in dem nächsten Schritt ohne Reinigung verwendet.

HPLC: t_{R} = 7,26 min. LC-MS: t_{R} = 12,57 min, m/z = 530,05 [M+H]⁺, 1059,16 [2M+H]⁺. ¹H NMR (DMSO-d⁶, 500 MHz): 12,70 (br, 1H), 10, 12 (s, 1H), 7,88 (m, 2H), 7,72-7,60 (m, 4H), 7,43-7,39 (m, 2H), 7,34-7,28 (m, 2H), 7,09 (m, 1H), 6,79 (m, 1H), 4,21-4,18 (m, 3H), 4,10-5,05 (m, 1H), 2,97-2,93 (m, 1H), 2,75-2,70 (m, 1H).

### b) Synthese von Fmoc-3-Iod-D-Tyr(MOM)-OMOM (13)

Fmoc-3-Iod-D-Tyr-OH **12** (9,5 g i.e. 8,62 g, 16,28 mmol ≡ 100 %) wurde in 120 ml DCM (wasserfrei) unter einer Argonatmosphäre suspendiert. DIPEA (5,673 ml, 32,57 mmol, 2 Äqu.) wurde zugegeben, was nach dem Rühren für 10 min bei Raumtemperatur zu einer gelben Lösung führte. TBAC1 (453 mg, 1,628 mmol, 0,1 Äqu.) wurde zugegeben, und das Gemisch wurde in einem eisgekühlten Wasserbad abgekühlt. Methoxymethylbromid (MOMBr) (2,658 ml, 32,57 mmol, 2 Äqu.), verdünnt in 30 ml DCM (wasserfrei), wurde tropfenweise über einen Tropftrichter unter einer Argonatmosphäre (Gasentwicklung) zugegeben. Nach der Zugabe wurde das Reaktionsgemisch unter Eiskühlung gerührt Nach einer Stunde wurde für weitere 18 h bei Raumtemperatur weitergerührt. Die DC (DCM/MeOH, 50:1) zeigte einen vollständigen Umsatz. 100 ml H₂O wurden zugegeben, und das Gemisch wurde bei Raumtemperatur kräftig gerührt. Nach 1 h wurden die Phasen in einem Scheidetrichter voneinander getrennt. Die wässerige Phase wurde mehrmals mit jeweils 150 ml DCM extrahiert. Die vereinigten organischen Phasen wurden mit 1 N HCl (2 x 150 ml) und Salzlösung (150 ml) gewaschen, über Na₂SO₄ getrocknet und filtriert (Filter Porengröße 4). Das Lösungsmittel wurde im Vakuum entfernt; der verbleibende Rest wurde im Hochvakuum getrocknet. Ausbeute: 11 g (109 %, quant.) eines weißen schaumartigen Feststoffes. Das Rohprodukt wurde im nächsten Schritt ohne Reinigung verwendet.

HPLC: t_{R} = 8,97 min. LC-MS: t_{R} = 14,76 min, m/z = 618,12 [M+H]⁺, 1235,32 [2M+H]⁺.

### c) Synthese von Fmoc-3-Iod-D-Tyr(MOM)-OH (1D)

Fmoc-3-Iod-D-Tyr(MOM)-OMOM **13** wurde in 140 ml THF (p.a.) gelöst. Während des Rührens wurde ein Gemisch aus 400 ml H₂O und 10 ml Pyridin zugegeben. Ca. 100 ml THF (p.a.) wurden zugegeben, bis ein klares Gemisch entstand. Unter kräftigem Rühren wurde das Gemisch in einem Ölbad (70 °C) zum Rückfluss erhitzt. Nach 64 h zeigte sich in der HPLC (214 nm) ein vollständiger Umsatz des Ausgangsmaterials (t_{R} = 8,96 min) zum Produkt Fmoc-3-Iod-D-Tyr(MOM)-OH. Das Lösungsmittel (THF) wurde im Vakuum verdampft. Das Gemisch wurde unter Eiskühlung mit 2 N HCl (ungefähr 120 ml) versetzt. Der pH der Lösung lag zwischen einem pH von 4 und einem pH von 5. Das Gemisch wurde mit DCM (3 x 150 ml) extrahiert, die vereinigten organischen Phasen wurden mit 0,5 N HCl (2 x 150 ml) und einer gesättigten Salzlösung (150 ml) gewaschen, über Na₂SO₄ getrocknet und filtriert. Das Lösungsmittel wurde im Vakuum verdampft, der verbleibende Rest wurde im Hochvakuum getrocknet. Ausbeute: 9,7 g (104 %, quant.) eines weißen schaumartigen Feststoffes. Das Rohprodukt wurde durch Säulenchromatographie gereinigt (Ausbeute: 4,6 g, Reinheit durch HPLC (214 nm): > 95 %).

m/z = 574,11 [M+H]⁺, 1147,26 [2M+H]⁺. ¹H-NMR (400 MHz, CDCl3) δ (ppm): 7,752 (d, 2H), 7,610 (s, 1H), 7,549 (m, 2H), 7,387 (t, 2H), 7,307 (m, 2H), 7,042(d, 1H), 6,960 (d, 2H), 5,185 (s, 2H), 4,697 (m, 1H), 4,444 (m, 1H), 4,336 (m, 1H), 4,201 (m, 1H), 3,483 (s, 3H), 3,131 (m, 1H), 3,004 (m, 1H).

### Beispiel 2

### Synthese von Boc-3-Iod-D-Tyr(MOM)-OH (5D)

Die Synthese von Boc-3-Iod-D-Tyr(MOM)-OH wurde, wie in Schema B2 beschrieben, durchgeführt: 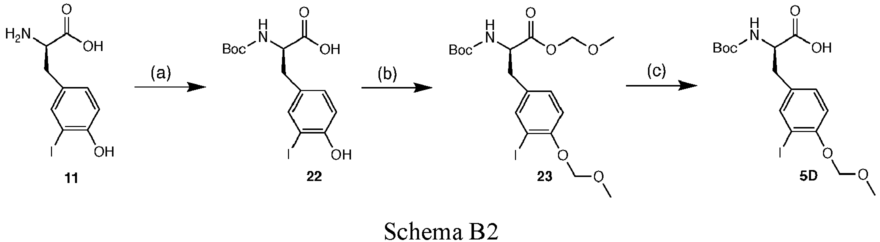 In Schritt (a) wird (*R*)-2-Amino-3-(4-hydroxy-3-iodphenyl)propionsäure **11** (die auch als 3-Iod-D-Tyrosin oder 3-Iod-D-Tyr-OH bezeichnet wird) mit Di-*tert*-butyldicarbonat (Boc₂O) unter Erhalt von (*R*)-2-((*tert*-Butoxycarbonyl)amino)-3-(4-hydroxy-3-iodphenyl)propionsäure **22** (die auch als Boc-3-Iod-D-Tyr-OH bezeichnet wird) umgesetzt. Die Umsetzung findet in einem Gemisch aus Wasser, Tetrahydrofuran und Triethylamin statt. In Schritt (b) wird Verbindung **22** dann mit Methoxymethylbromid (CH₃-O-CH₂-Br) zu Methoxymethyl-(*R*)-2-((*tert*-butoxycarbonyl)amino)-3-(3-iod-4-(methoxymethoxy)phenyl)propanoat **23** (das auch als Boc-3-Iod-D-Tyr(MOM)-OMOM bezeichnet wird) umgesetzt. Die Umsetzung findet in Dichlormethan (DCM) in Gegenwart von Diisopropylethylamin (DIPEA) und Tetrabutylammoniumchlorid (TBACl) statt. In Schritt (c) wird Verbindung 23 dann zur Zielverbindung 5D umgesetzt. Die Umsetzung erfolgt in einem Gemisch aus Tetrahydrofuran (THF), Wasser und Pyridin.

### a) Synthese von Boc-3-Iod-D-Tyr-OH (22)

3-Iod-D-Tyr-OH 11 (16,28 mmol) wurde in 150 ml einer Mischung aus THF/H₂O (1 : 1) gelöst und TEA (4,44 ml, 32,56 mmol, 2 Äqu.) hinzugetropft. Das Gemisch wurde auf Eis auf 0 °C gekühlt. Boc₂O (3,63 ml, 17,9 mmol, 1,1 Äqu.) wurde im Wasserbad bei 30 °C geschmolzen und anschließend in 20 ml THF gelöst. Die Lösung wurde in einen Tropftrichter überführt und über einen Zeitraum von 30 Minuten zugetropft. Nach einer Stunde wurde das Eisbad entfernt und das Reaktionsgemisch über Nacht bei Raumtemperatur gerührt. Die Vollständigkeit des Umsatzes wurde mittels HPLC kontrolliert. Das THF wurde im Vakuum entfernt. Die wässrige Lösung wurde mit 1 M HCl auf pH 3-4 eingestellt und dreimal mit je 150 ml Ethylacetat extrahiert. Die vereinigten organischen Phasen wurden über Natriumsulfat getrocknet und das Lösungsmittel im Vakuum entfernt. Das Produkt wurde im Hochvakuum getrocknet. Die Reinheit des Syntheseprodukts (Boc-3-Iod-D-Tyr-OH **22)** wurde mittels HPLC ermittelt (> 95 %).

### b) Synthese von Boc-3-Iod-D-Tyr(MOM)-OMOM (23)

Boc-3-Iod-D-Tyr-OH **22** (16,28 mmol) wurde in 120 ml trockenem DCM gelöst. Es wurden DIPEA (5,67 ml, 32,56 mmol, 2 Äqu.) und Tetrabutylammoniumchlorid (0,453 g, 1,63 mmol, 0,1 Äqu.) hinzugegeben. Eine Lösung von Methoxymethylbromid (2,657 ml, 32,56 mmol, 2 Äqu.) in 30 ml DCM (wasserfrei) wurde über einen Zeitraum von 30 Minuten zu einer eisgekühlten Lösung von Boc-3-Iod-D-Tyr-OH langsam zugetropft. Nach einer Stunde wurde das Eisbad entfernt und anschließend über Nacht bei Raumtemperatur gerührt. Nach Zugabe von Wasser und anschließender Abtrennung und Trocknung der organischen Phase wurde die organische Phase im Vakuum verdampft. Die Vollständigkeit des Umsatzes wurde mittels HPLC kontrolliert. Das Produkt Boc-3-Iod-D-Tyr(MOM)-OMOM **23** wurde mittels HPLC identifiziert (> 95 %).

### c) Synthese von Boc-3-Iod-D-Tyr(MOM)-OH (5D)

Boc-I-D-Tyr(MOM)-OMOM wurde in 20 ml THF gelöst. Es wurden 20 ml einer 2 M Lösung von LiOH in Wasser hinzugegeben und 2 Stunden bei Raumtemperatur gerührt. Das THF wurde im Vakuum entfernt. Es wurden 300 ml DCM und 150 ml einer 5%-igen KHSO4-Lösung hinzugegeben und 5 Minuten gerührt. Nach Phasentrennung wurde die wässrige Phase einmal mit 150 ml DCM extrahiert. Die vereinigten organischen Phasen wurden über Na2SO4 getrocknet und das Lösungsmittel im Vakuum entfernt. Das erhaltene Produkt wurde lyophilisiert.

¹H-NMR (400 MHz, CDCl3) δ (ppm): 7,619 (s, 1H), 7,103 (d, 1H), 6,993 (d, 1H), 5,216 (s, 2H), 4,968; 4,54 (m, 1H), 3,504 (s, 3H), 3,138 (m, 1H), 2,993 (m, 1H), 1,440 (s, 9H).

### Beispiel 3

### a) Synthese von Tripeptiden

Zum Nachweis der verbesserten Kopplungseigenschaften der erfindungsgemäßen Verbindungen der allgemeinen Formel I-A wurden Tripeptide hergestellt. Die hergestellten Tripeptide sind in Tabelle 2 gezeigt, wobei Ac Acetyl, Me Methyl, Amb Aminomethylbenzoyl und Pbf eine 2,2,4,6,7-Pentamethyldihydrobenzofuran-5-sulfonyl-Gruppe bezeichnet.

Für die Herstellung der erfindungsgemäßen Tripeptide **P1** und **P2** wurde entweder Fmoc-3-Iod-D-Tyr(MOM)-OH (Verbindung **1D**) oder Boc-3-Iod-D-Tyr(MOM)-OH (Verbindung **5D)** eingesetzt. Außerdem wurden zu Vergleichszwecken die Tripeptide **V1** und **V2** hergestellt. Die Tripeptide **V1** und **V2** unterscheiden sich von den Tripetiden **P1** und **P2** durch den Schutz der phenolischen Hydroxygruppe. Bei den Tripetiden **P1** und **P2** ist die phenolische Hydroxygruppe durch eine -CH₂-O-CH₃Gruppe (MOM) geschützt, während sie bei den Tripeptiden **V1** und **V2** nicht geschützt ist.

**Tabelle 2**

| Verbindung | Struktur | Bezeichnung (Sequenz) |
|---|---|---|
| **P1** | 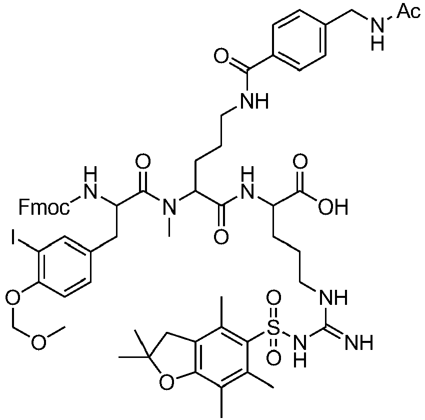 | Fmoc-3 -Iod-D-Tyr(MOM)-*N*-Me-D-Orn(Amb-Ac)-Arg(Pbf)-OH |
| **V1** | 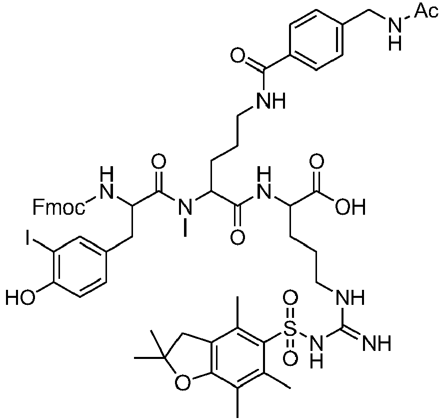 | Fmoc-3-Iod-D-Tyr-*N*-Me-D-Orn(Amb-Ac)-Arg(Pbf)-OH |
| **P2** | 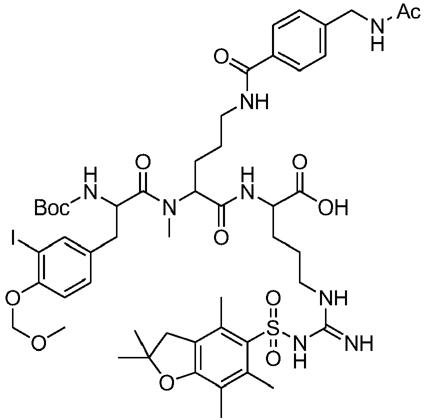 | B oc-3 -Iod-D-Tyr(MOM)-*N*-Me-D-Orn(Amb-Ac)-Arg(Pbf)-OH |
| **V2** | 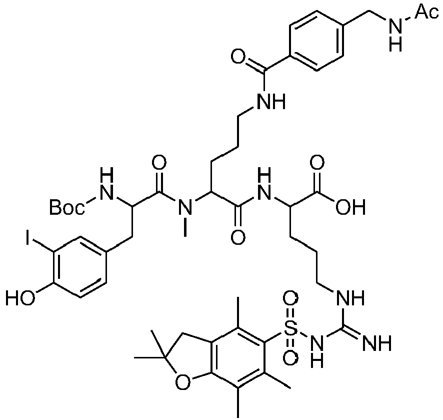 | Boc-3-Iod-D-Tyr-*N*-Me-DOrn(Amb-Ac)-Arg(Pbf)-OH |

Die Tripeptide wurden mit Hilfe der von Merrifield entwickelten Fmoc/tBu-Strategie auf einem Chlortrityl-Harz, das auch als "Barlos-Harz" bezeichnet wird, hergestellt (Barlos, K., et al., Darstellung geschützter Peptid-Fragmente unter Einsatz substituierter Triphenylmethylharze, Tetrahedron Letters, 1989, 30(30), S. 3943-3946). Dies ermöglicht die Abspaltung voll geschützter Peptid-Fragmente mit Hilfe schwach saurer Verbindungen wie Hexafluorisopropanol (HFIP). Die Kupplung aller aminosäureartigen Komponenten erfolgte mit Hilfe von Diisopropylcarbodiimid (DIC) und Hydroxyiminocyanessigsäureethylester (Oxyma). Die Abspaltung der Fmoc-Schutzgruppen erfolgte mit 20 % Piperidin in DMF. Die Abspaltung des Peptids vom Harz erfolgte mit 20 % 1,1,1,3,3,3-Hexafluorpropan-2-ol (HFIP) in DCM.

### b) Vergleichsversuche

Die erfindungsgemäßen Tripeptide **P1** und **P2** und die Vergleichszwecken dienenden Tripeptide **V1** und **V2** wurden unter Verwendung von Diisopropylcarbodiimid (DIC) und Hydroxyiminocyanessigsäureethylester (Oxyma) innerhalb von 60 min gekuppelt.

Zur Herstellung des Tripeptids **P1** wurde Fmoc-3-Iod-D-Tyr(MOM)-OH **(1D)** an H-*N*-Me-D-Orn(Amb-Ac)-Arg(Pbf)-Chlortritylharz gekuppelt. Die Kupplung wurde kinetisch verfolgt. Die Ergebnisse sind in Tabelle 3 gezeigt.

**Tabelle 3**

| Synthese von Fmoc-3-Iod-D-Tyr(MOM)-*N*-Me-D-Orn(Amb-Ac)-Arg(Pbf)-OH (**P1**) aus Fmoc-3-Iod-D-Tyr(MOM)-OH **(1D)** und H-*N*-Me-D-Orn(Amb-Ac)-Arg(Pbf)-Chlortritylharz | | | |
|---|---|---|---|
| Zeit (min) | Edukt* (%) | Produkt **P1** (%)** | Nebenprodukte (%)*** |
| 0 | 100 | 0 | 0 |
| 15 | 73 | 26 | 0 |
| 60 | 40 | 60 | 0 |
| 120 | 14 | 86 | 0 |
| 720 | 6 | 94 | 0 |

| | | | |
|---|---|---|---|
| *Edukt ist H-*N*-Me-D-Orn(Amb-Ac)-Arg(Pbf)-OH, da die Abnahme von Fmoc-3-Iod-D-Tyr(MOM)-OH (nur mit einigem Aufwand bestimmt werden kann. ** Fmoc-3-Iod-D-Tyr(MOM)-*N*-Me-D-Orn(Amb-Ac)-Arg(Pbf)-OH **(P1)** *** Es wurde nicht näher bestimmt, um welche Nebenprodukte es sich handelt. | | | |

Zur Herstellung des Tripeptids **V1** wurde Fmoc-3-Iod-D-Tyr-OH an H-*N*-Me-D-Orn(Amb-Ac)-Arg(Pbf)-Chlortritylharz gekuppelt. Die Kupplung wurde kinetisch verfolgt. Die Ergebnisse sind in Tabelle 4 gezeigt.

**Tabelle 4**

| Synthese von Fmoc-3-Iod-D-Tyr-*N*-Me-D-Orn(Amb-Ac)-Arg(Pbf)-OH (**V1**) aus Fmoc-3-Iod-D-Tyr-OH und H-*N*-Me-D-Orn(Amb-Ac)-Arg(Pbf)-Chlortritylharz | | | |
|---|---|---|---|
| Zeit (min) | Edukt* (%) | Produkt **V1** (%)** | Nebenprodukte (%)*** |
| 0 | 100 | 0 | 0 |
| 15 | 81 | 14,2 | 4,7 |
| 60 | 65,8 | 21,9 | 12,2 |
| 120 | 59,8 | 26 | 14,1 |
| 720 | 51,5 | 29 | 19,5 |

| | | | |
|---|---|---|---|
| *Edukt ist H-N-Me-D-Orn(Amb-Ac)-Arg(Pbf)-OH, da die Abnahme von Fmoc-3-Iod-D-Tyr-OH nur mit hohem Aufwand bestimmt werden kann. ** Fmoc-3-Iod-D-Tyr-N-Me-D-Orn(Amb-Ac)-Arg(Pbf)-OH **(V1)** *** Es wurde nicht näher bestimmt, um welche Nebenprodukte es sich handelt. | | | |

Zur Herstellung des Tripeptids **P2** wurde Boc-3-Iod-D-Tyr(MOM)-OH **(5D)** an H-*N*-Me-D-Orn(Amb-Ac)-Arg(Pbf)-Chlortritylharz gekuppelt. Die Kupplung wurde kinetisch verfolgt. Die Ergebnisse sind in Tabelle 5 gezeigt.

**Tabelle 5**

| Synthese von Boc-3-Iod-D-Tyr(MOM)-*N*-Me-D-Orn(Amb-Ac)-Arg(Pbf)-OH **(P2)** aus Boc-3-Iod-D-Tyr(MOM)-OH und H-*N*-Me-D-Orn(Amb-Ac)-Arg(Pbf)-Chlortritylharz | | | |
|---|---|---|---|
| Zeit (min) | Edukt* (%) | Produkt **P2** (%)** | Nebenprodukte (%)*** |
| 0 | 100 | 0 | 0 |
| 15 | 87,3 | 12,17 | 0 |
| 60 | 37,16 | 62,84 | 0 |
| 120 | 13,37 | 86,63 | 0 |
| 720 | 0,68 | 99,32 | 0 |

| | | | |
|---|---|---|---|
| *Edukt ist H-*N*-Me-D-Orn(Amb-Ac)-Arg(Pbf)-OH, da die Abnahme von Boc-3-Iod-D-Tyr(MOM)-OH nur mit einigem Aufwand bestimmt werden kann. ** Boc-3-Iod-D-Tyr(MOM)-*N*-Me-D-Orn(Amb-Ac)-Arg(Pbf)-OH **(P2)** *** Es wurde nicht näher bestimmt, um welche Nebenprodukte es sich handelt. | | | |

Zur Herstellung des Tripeptids **V2** wurde Boc-3-Iod-D-Tyr-OH an H-*N*-Me-DOrn(Amb-Ac)-Arg(Pbf)-Chlortritylharz gekuppelt. Die Kupplung wurde kinetisch verfolgt. Die Ergebnisse sind in Tabelle 6 gezeigt.

**Tabelle 6**

| Synthese von Boc-3-Iod-D-Tyr-N-Me-D-Orn(Amb-Ac)-Arg:(Pbf)-OH (**V2**) aus Boc-3-Iod-D-Tyr-OH und H-*N*-Me-D-Orn(Amb-Ac)-Arg(Pbf)-Chlortritylharz | | | |
|---|---|---|---|
| Zeit (min) | Edukt* (%) | Produkt **V2** (%)** | Nebenprodukte (%)*** |
| 0 | 100 | 0 | 0 |
| 15 | 93,2 | 5,7 | 1,06 |
| 60 | 73,3 | 16,1 | 10,6 |
| 120 | 65,2 | 19,8 | 15 |
| 720 | 53,74 | 18,56 | 27,7 |

| | | | |
|---|---|---|---|
| *Edukt ist H-*N*-Me-D-Orn(Amb-Ac)-Arg(Pbf)-OH, da die Abnahme von Boc-3-Iod-D-Tyr-OH nur mit einigem Aufwand bestimmt werden kann. ** Boc-3-Iod-D-Tyr-*N*-Me-D-Orn-(Amb-Ac)-Arg(Pbf)-OH *** Es wurde nicht näher bestimmt, um welche Nebenprodukte es sich handelt. | | | |

Die Herstellung der erfindungsgemäßen Tripeptide **P1** und **P2** und der Vergleichszwecken dienenden Tripeptide **V1** und **V2** zeigt, dass sowohl die Verwendung von Fmoc-3-Iod-D-Tyr(MOM)-OH **(1D)** als auch von Boc-3-Iod-D-Tyr(MOM)-OH (**5D**) zu der Zielverbindung in hoher Reinheit und Ausbeute führt. Die Verwendung der in der Seitenkette ungeschützten Iod-Tyrosinderivate führte zu einer erheblich verminderten Ausbeute bzw. der Ausbildung nicht näher spezifizierter Nebenprodukte. Die Tripeptide **P1** und **P2** belegen die erhöhte Effizienz der Peptidsynthese, die sich aus der Verwendung der erfindungsgemäßen Tyrosinderivate mit geschützter phenolischer Hydroxyfunktion ergibt.

### Beispiel 4

### Synthese von Pentixather

Pentixather konnte mit großer Effizienz mit Hilfe der Aminosäure Fmoc-3-Iod-D-Tyr(MOM)-OH **(1D)** hergestellt werden, während die Verwendung der ungeschützten Aminosäure Fmoc-3-Iod-D-Tyr-OH zu keinem oder nur einem geringen Umsatz führte.

### Beispiel 5

### Synthese von PSMAI&T

Die Synthese von der Verbindung Glu-CO-Lys[(Sub)DLys-DPhe-DTyr(3I)-DOTAGA]trifluoracetat (PSMAI&T) (Wirtz, M., et al., Synthesis and in vitro and in vivo evaluation of urea-based PSMA inhibitors with increased lipophilicity. EJNMMI Research, 2018. 8(1): p. 84) verlief analog zur Synthese von Pentixather mit größerer Effizienz und erheblich verbesserter Reinheit des Endprodukts bei Verwendung von Boc-3-Iod-D-Tyrosin(MOM)-OH **(1D)** anstatt des ungeschützten Derivats.

### Zitierte Literatur

1. Sadri, K., et al., Synthesis and biodistribution studies of iodine-131 D-amino acid YYKpeptide as apotential therapeutic agent for labeling an anti-CD20 antibody. 2009. 52(7): p. 289-294.
2. Hallaba, E., H. E1-Asrag, and Y. Abou Zeid, 131I-labelling of tyrosine by iodine monochloride. The International Journal of Applied Radiation and Isotopes, 1970. 21(2): p. 107-110.
3. Martin, E.B., et al., Evaluation of the effect of D-amino acid incorporation into amyloid-reactive peptides. Journal of translational medicine, 2017. 15(1): p. 247-247.
4. Assoian, R.K., et al., Iodotyrosylation of peptides using tertiary-butyloxycarbonyl-l-[125I]iodotyrosine N-hydroxysuccinimide ester. Analytical Biochemistry, 1980. 103(1): p. 70-76.
5. Schottelius, M., et al., [(177)Lu]pentixather: Comprehensive Preclinical Characterization of a First CXCR4-directedEndoradiotherapeutic Agent. Theranostics, 2017. 7(9): p. 2350-2362.
6. Brogsitter, C., et al., Twins in spirit part II: DOTATATE andhigh-affinity DOTA-TATE-the clinical experience. European journal of nuclear medicine and molecular imaging, 2014. 41.
7. Weineisen, M., et al., 68Ga- and 177Lu-Labeled PSMA I&T: Optimization of a PSMA-Targeted Theranostic Concept and First Proof-of-Concept Human Studies. J Nucl Med, 2015. 56(8): p. 1169-76.
8. A, W.M., IODINATED INSULINANALOGUES WITH FORESHORTENED SIG-NALING. 14.12.2016.
9. Schottelius, M., et al., An optimized strategy for the mild and efficient solution phase iodination of tyrosine residues in bioactive peptides. Tetrahedron Letters, 2015. 56(47): p. 6602-6605.
10. Steer, A.M., et al., A direct route for the preparation of Fmoc/OtBu protected iodotyrosine. Tetrahedron Letters, 2018. 59(27): p. 2644-2646.
11. White, J.D. and J.C. Amedio, Total synthesis of geodiamolide A, a novel cyclodepsipeptide of marine origin. The Journal of Organic Chemistry, 1989. 54(4): p. 736-738.
12. Ishiwata, H., et al., Total Synthesis of Doliculide, a Potent Cytotoxic Cyclodepsipeptide from the Japanese Sea Hare Dolabella auricularia. The Journal of Organic Chemistry, 1994. 59(17): p. 4712-4713.
13. Pedersen, M.H.F. and L. Martiny, Homogeneous deuteriodeiodination of iodinated tyrosine in angiotensin-I using synthesized triethyl[2H]silane and Pd(0). 2011. 54(4): p. 191-195.

## Patentansprüche

1. Verbindung der allgemeinen Formel I 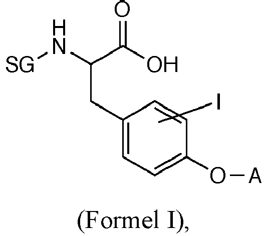 worin
A aus der Gruppe ausgewählt ist, die aus einer unverzweigten oder verzweigten Alkylgruppe mit 1 bis 12 Kohlenstoffatomen, einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R⁶)(O-R⁷)-Gruppe, einer -R¹-O-C(O)-O-R⁸-Gruppe besteht;
SG eine Schutzgruppe ist;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist; und
R⁹ ein zweiwertiger Kohlenwasserstoffrest mit 1 bis 12 Kohlenstoffatomen ist.

2. Verbindung nach Anspruch 1, **dadurch gekennzeichnet, dass** sie eine Verbindung der allgemeinen Formel I-A 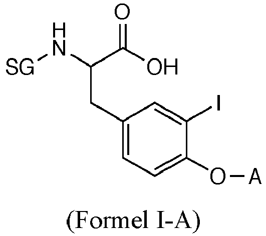 ist, worin A und SG die in Anspruch 1 genannten Bedeutungen aufweisen.

3. Verbindung nach einem der vorstehenden Ansprüche, **dadurch gekennzeichnet, dass** SG aus der Gruppe ausgewählt ist, die aus einer Fluorenylmethylenoxycarbonyl-Gruppe (Fmoc), einer *tert*-Butoxycarbonyl-Gruppe (Boc) und einer Benzyloxycarbonyl-Gruppe besteht.

4. Verbindung nach einem der vorstehenden Ansprüche, **dadurch gekennzeichnet, dass** A aus der Gruppe ausgewählt ist, die aus einer unverzweigten oder verzweigten Alkylgruppe mit 1 bis 12 Kohlenstoffatomen, einer -R¹-O-R²-Gruppe, einer - R¹-Si(R³R⁴R⁵)-Gruppe und einer -C(O)-O-R⁹-Si(R³R⁴R⁵)-Gruppe besteht, wobei R¹, R², R³, R⁴, R⁵ und R⁹ die in Anspruch 1 angegebenen Bedeutungen haben.

5. Verbindung nach einem der vorstehenden Ansprüche, **dadurch gekennzeichnet, dass** A aus der Gruppe ausgewählt ist, die aus einer Alkylgruppe mit 1 bis 6 Kohlenstoffatomen; einer -R¹-O-R²-Gruppe, in der R¹ eine Alkylengruppe mit 1 bis 6 Kohlenstoffatomen ist und R² eine unverzweigte oder verzweigte Alkylgruppe mit 1 bis 6 Kohlenstoffatomen ist; einer -R¹-Si(R³R⁴R⁵)-Gruppe, in der R¹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; und einer -C(O)-O-R⁹-Si(R³R⁴R⁵)-Gruppe, in der R⁹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; besteht.

6. Verbindung nach einem der vorstehenden Ansprüche, **dadurch gekennzeichnet, dass** A aus der Gruppe ausgewählt ist, die aus einer Alkylgruppe mit 1 bis 6 Kohlenstoffatomen; einer -R¹-O-R²-Gruppe, in der R¹ eine Alkylengruppe mit 1 bis 4 Kohlenstoffatomen ist und R² eine unverzweigte oder verzweigte Alkylgruppe mit 1 bis 6 Kohlenstoffatomen ist; einer -R¹-Si(R³R⁴R⁵)-Gruppe, in der R¹ eine Alkylengruppe mit 1 bis 4 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; und einer -C(O)-O-R⁹-Si(R³R⁴R⁵)-Gruppe, in der R⁹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; besteht.

7. Verbindung nach einem der vorstehenden Ansprüche, **dadurch gekennzeichnet, dass** sie
2-((((9*H*-Fluoren-9-yl)methoxy)carbonyl)amino)-3-(3-iod-4-(methoxy-methoxy)phenyl)propionsäure,
2-((((9*H*-Fluoren-9-yl)methoxy)carbonyl)amino)-3-(3-iod-4-(((2-(trimethylsilyl)ethoxy)carbonyl)oxy)phenyl)propionsäure,
2-((((9*H*-Fluoren-9-yl)methoxy)carbonyl)amino)-3-(4-(2-(*tert*-butyldiphenylsilyl)ethoxy)-3-iodphenyl)propionsäure,
2-((((9*H*-Fluoren-9-yl)methoxy)carbonyl)amino)-3-(4-(*tert*-butoxy)-3-iodphenyl)propionsäure ist.

8. Verbindung nach einem der Ansprüche 1 bis 6, **dadurch gekennzeichnet, dass** sie
2-((*tert*-Butoxycarbonyl)amino)-3-(3-iod-4-(methoxymethoxy)phenyl)propionsäure,
2-((*tert*-Butoxycarbonyl)amino)-3-(3-iod-4-(((2-(trimethylsilyl)ethoxy)carbonyl)oxy)phenyl)propionsäure,
2-((*tert*-Butoxycarbonyl)amino)-3-(4-(2-(*tert*-butyldiphenylsilyl)ethoxy)-3-iodphenyl)propionsäure, oder
2-((*tert*-Butoxycarbonyl)amino)-3-(4-(*tert*-butoxy)-3-iodphenyl)propionsäure ist.

9. Verfahren zur Herstellung einer Verbindung gemäß einem der Ansprüche 1 bis 8, **dadurch gekennzeichnet, dass** eine Verbindung der allgemeinen Formel II 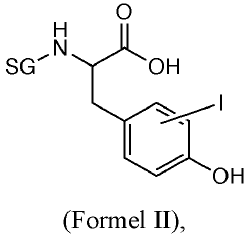 worin SG eine Schutzgruppe ist,
mit einer Verbindung der allgemeinen Formel X-A, worin
X Halogen oder Ammonium ist und
A aus der Gruppe ausgewählt ist, die aus einer unverzweigten oder verzweigten Alkylgruppe mit 1 bis 12 Kohlenstoffatomen, einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R₆)(O-R⁷)-Gruppe, einer -R¹-O-C(O)-O-R⁸-Gruppe besteht;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist; und
R⁹ ein zweiwertiger Kohlenwasserstoffrest mit 1 bis 12 Kohlenstoffatomen ist, zu einer Verbindung der allgemeinen Formel I 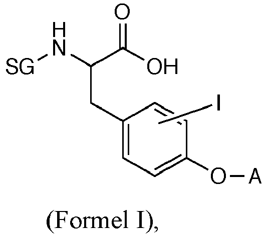 worin A und SG die im Zusammenhang mit Formel II angegebenen Bedeutungen aufweisen, umgesetzt wird.

10. Verfahren nach Anspruch 9, **dadurch gekennzeichnet, dass** die Verbindung der allgemeinen Formel II mit einer Verbindung der allgemeinen der Formel X-A unter Erhalt einer Verbindung der allgemeinen Formel III 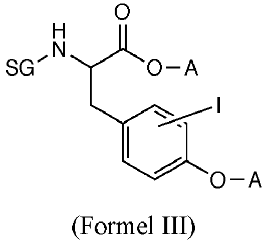 umgesetzt wird und die Verbindung der allgemeinen Formel III anschließend zu einer Verbindung der allgemeinen Formel I umgesetzt wird.

11. Verfahren nach Anspruch 9 oder Anspruch 10, **dadurch gekennzeichnet, dass** die Verbindung der allgemeinen Formel II aus einer Verbindung der allgemeinen Formel IV 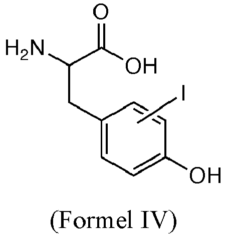 durch Einführung einer Schutzgruppe SG an der Aminogruppe der Verbindung der allgemeinen Formel IV hergestellt wird.

12. Verwendung einer Verbindung gemäß einem der Ansprüche 1 bis 8 zur Herstellung eines Peptides.

13. Verwendung nach Anspruch 12, **dadurch gekennzeichnet, dass** das Peptid eine Verbindung der allgemeinen Formel IX 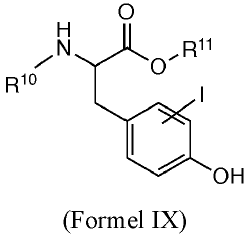 ist, worin
R¹⁰ Wasserstoff oder eine oder mehrere Aminosäureeinheiten ist; und
R¹¹ Wasserstoff oder eine oder mehrere Aminosäureeinheiten ist, mit der Maßgabe, dass, wenn R¹⁰ Wasserstoff ist, R¹¹ nicht Wasserstoff ist und dass, wenn R¹¹ Wasserstoff ist, R¹⁰ nicht Wasserstoff ist.

14. Verwendung nach Anspruch 12 oder Anspruch 13, **dadurch gekennzeichnet, dass** eine Verbindung der allgemeinen Formel VIII 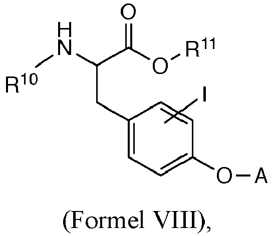 worin
A aus der Gruppe ausgewählt ist, die aus einer unverzweigten oder verzweigten Alkylgruppe mit 1 bis 12 Kohlenstoffatomen, einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R⁶)(O-R⁷)-Gruppe, einer -R¹-O-C(O)-O-R⁸-Gruppe besteht;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist;
R⁹ ein zweiwertiger Kohlenwasserstoffrest mit 1 bis 12 Kohlenstoffatomen ist; und
R¹⁰ und R¹¹ die in Zusammenhang mit der Verbindung der allgemeinen Formel IX angegebenen Bedeutungen haben;
zu einer Verbindung der allgemeinen Formel XI umgesetzt wird.

15. Verwendung nach Anspruch 14, **dadurch gekennzeichnet, dass** die Umsetzung im sauren Bereich erfolgt.

## Geänderte Patentansprüche

### Geänderte Patentansprüche gemäss Regel 137(2) EPÜ.

1. Verbindung der allgemeinen Formel I 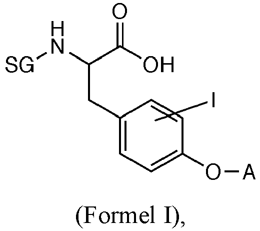 worin
A aus der Gruppe ausgewählt ist, die aus einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R⁶)(O-R⁷)-Gruppe, einer -R¹-O-C(O)-O-R⁸-Gruppe besteht;
SG eine Schutzgruppe ist;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist; und
R⁹ ein zweiwertiger Kohlenwasserstoffrest mit 1 bis 12 Kohlenstoffatomen ist.

2. Verbindung nach Anspruch 1, **dadurch gekennzeichnet, dass** sie eine Verbindung der allgemeinen Formel I-A 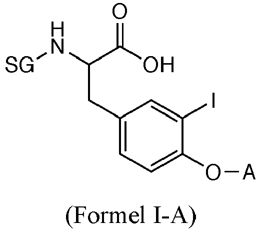 ist, worin A und SG die in Anspruch 1 genannten Bedeutungen aufweisen.

3. Verbindung nach einem der vorstehenden Ansprüche, **dadurch gekennzeichnet, dass** SG aus der Gruppe ausgewählt ist, die aus einer Fluorenylmethylenoxycarbonyl-Gruppe (Fmoc), einer *tert*-Butoxycarbonyl-Gruppe (Boc) und einer Benzyloxycarbonyl-Gruppe besteht.

4. Verbindung nach einem der vorstehenden Ansprüche, **dadurch gekennzeichnet, dass** A aus der Gruppe ausgewählt ist, die aus einer -R¹-O-R²-Gruppe, einer -R¹-Si(R³R⁴R⁵)-Gruppe und einer -C(O)-O-R⁹-Si(R³R⁴R⁵)-Gruppe besteht, wobei R¹, R², R³, R⁴, R⁵ und R⁹ die in Anspruch 1 angegebenen Bedeutungen haben.

5. Verbindung nach einem der vorstehenden Ansprüche, **dadurch gekennzeichnet, dass** A aus der Gruppe ausgewählt ist, die aus einer -R¹-O-R²-Gruppe, in der R¹ eine Alkylengruppe mit 1 bis 6 Kohlenstoffatomen ist und R² eine unverzweigte oder verzweigte Alkylgruppe mit 1 bis 6 Kohlenstoffatomen ist; einer -R¹-Si(R³R⁴R⁵)-Gruppe, in der R¹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; und einer -C(O)-O-R⁹- Si(R³R⁴R⁵)-Gruppe, in der R⁹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; besteht.

6. Verbindung nach einem der vorstehenden Ansprüche, **dadurch gekennzeichnet, dass** A aus der Gruppe ausgewählt ist, die aus einer -R¹-O-R²-Gruppe, in der R¹ eine Alkylengruppe mit 1 bis 4 Kohlenstoffatomen ist und R² eine unverzweigte oder verzweigte Alkylgruppe mit 1 bis 6 Kohlenstoffatomen ist; einer -R¹-Si(R³R⁴R⁵)-Gruppe, in der R¹ eine Alkylengruppe mit 1 bis 4 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; und einer -C(O)-O-R⁹- Si(R³R⁴R⁵)-Gruppe, in der R⁹ eine Alkylengruppe mit 1 bis 6 Kohlenstoffatomen ist und R³, R⁴ und R⁵ unabhängig voneinander jeweils eine unverzweigte oder verzweigte Alkylgruppe mit 1 bis 6 Kohlenstoffatomen oder eine Arylgruppe sind; besteht.

7. Verbindung nach einem der vorstehenden Ansprüche, **dadurch gekennzeichnet, dass** sie
2-((((9*H*-Fluoren-9-yl)methoxy)carbonyl)amino)-3-(3-iod-4-(methoxy-methoxy)phenyl)propionsäure,
2-((((9*H*-Fluoren-9-yl)methoxy)carbonyl)amino)-3-(3-iod-4-(((2-(trimethylsilyl)ethoxy)carbonyl)oxy)phenyl)propionsäure oder
2-((((9*H*-Fluoren-9-yl)methoxy)carbonyl)amino)-3-(4-(2-(*tert*-butyldiphenylsilyl)ethoxy)-3-iodphenyl)propionsäure ist.

8. Verbindung nach einem der Ansprüche 1 bis 6, **dadurch gekennzeichnet, dass** sie
2-((*tert*-Butoxycarbonyl)amino)-3-(3-iod-4-(methoxymethoxy)phenyl)propionsäure,
2-((*tert*-Butoxycarbonyl)amino)-3-(3-iod-4-(((2-(trimethylsilyl)ethoxy)carbonyl)oxy)phenyl)propionsäure oder
2-((*tert*-Butoxycarbonyl)amino)-3-(4-(2-(*tert*-butyldiphenylsilyl)ethoxy)-3-iodphenyl)propionsäure ist.

9. Verfahren zur Herstellung einer Verbindung gemäß einem der Ansprüche 1 bis 8, **dadurch gekennzeichnet, dass** eine Verbindung der allgemeinen Formel II 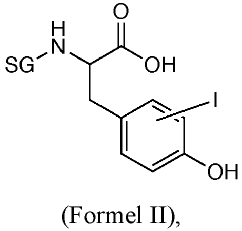 worin SG eine Schutzgruppe ist,
mit einer Verbindung der allgemeinen Formel X-A, worin
X Halogen oder Ammonium ist und
A aus der Gruppe ausgewählt ist, die aus einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R⁶)(O-R⁷)-Gruppe, einer -R¹-O-C(O)-O-R⁸-Gruppe besteht;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist; und
R⁹ ein zweiwertiger Kohlenwasserstoffrest mit 1 bis 12 Kohlenstoffatomen ist,
zu einer Verbindung der allgemeinen Formel I 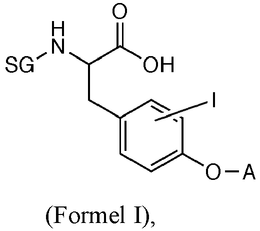 worin A und SG die im Zusammenhang mit Formel II angegebenen Bedeutungen aufweisen, umgesetzt wird.

10. Verfahren nach Anspruch 9, **dadurch gekennzeichnet, dass** die Verbindung der allgemeinen Formel II mit einer Verbindung der allgemeinen der Formel X-A unter Erhalt einer Verbindung der allgemeinen Formel III 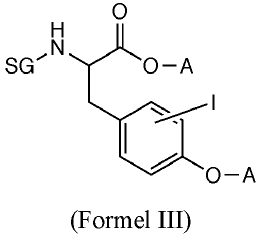 umgesetzt wird und die Verbindung der allgemeinen Formel III anschließend zu einer Verbindung der allgemeinen Formel I umgesetzt wird.

11. Verfahren nach Anspruch 9 oder Anspruch 10, **dadurch gekennzeichnet, dass** die Verbindung der allgemeinen Formel II aus einer Verbindung der allgemeinen Formel IV 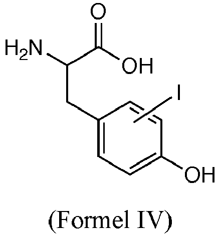 durch Einführung einer Schutzgruppe SG an der Aminogruppe der Verbindung der allgemeinen Formel IV hergestellt wird.

12. Verwendung einer Verbindung gemäß einem der Ansprüche 1 bis 8 zur Herstellung eines Peptides.

13. Verwendung nach Anspruch 12, **dadurch gekennzeichnet, dass** das Peptid eine Verbindung der allgemeinen Formel IX 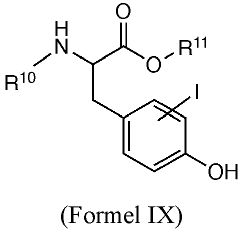 ist, worin
R¹⁰ Wasserstoff oder eine oder mehrere Aminosäureeinheiten ist; und
R¹¹ Wasserstoff oder eine oder mehrere Aminosäureeinheiten ist,
mit der Maßgabe, dass, wenn R¹⁰ Wasserstoff ist, R¹¹ nicht Wasserstoff ist und dass, wenn R¹¹ Wasserstoff ist, R¹⁰ nicht Wasserstoff ist.

14. Verwendung nach Anspruch 12 oder Anspruch 13, **dadurch gekennzeichnet, dass** eine Verbindung der allgemeinen Formel VIII 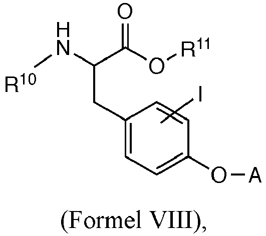 worin
A aus der Gruppe ausgewählt ist, die aus einer -R¹-O-R²-Gruppe, einer -R¹-Si(R³R⁴R⁵)-Gruppe, einer -R¹-O-Si(R³R⁴R⁵)-Gruppe, einer -C(O)-O-R⁹-Si(R³R⁴R⁵)-Gruppe, einer -CH(O-R⁶)(O-R⁷)-Gruppe, einer -R¹-CH(O-R⁶)(O-R⁷)-Gruppe, einer -R¹-O-C(O)-O-R⁸-Gruppe besteht;
R¹ ein zweiwertiger Kohlenwasserstoffrest mit 1 bis 12 Kohlenstoffatomen ist;
R² ein einwertiger Kohlenwasserstoffrest mit 1 bis 12 Kohlenstoffatomen ist;
R³, R⁴ und R⁵ unabhängig voneinander jeweils ein einwertiger Kohlenwasserstoffrest mit 1 bis 12 Kohlenstoffatomen sind;
R⁶ und R⁷ unabhängig voneinander jeweils ein einwertiger Kohlenwasserstoffrest mit 1 bis 12 Kohlenstoffatomen sind;
R⁸ ein einwertiger Kohlenwasserstoffrest mit 1 bis 12 Kohlenstoffatomen ist;
R⁹ ein zweiwertiger Kohlenwasserstoffrest mit 1 bis 12 Kohlenstoffatomen ist; und
R¹⁰ und R¹¹ die in Zusammenhang mit der Verbindung der allgemeinen Formel IX angegebenen Bedeutungen haben;
zu einer Verbindung der allgemeinen Formel IX umgesetzt wird.

15. Verwendung nach Anspruch 14, **dadurch gekennzeichnet, dass** die Umsetzung im sauren Bereich erfolgt.
